# EUROPEAN PATENT APPLICATION

(11) **EP 1 905 841 A1**
(43) Date of publication of application: **02.04.2008**
(21) Application number: 06020014.4
(22) Date of filing: 25.09.2006
(51) Int. Cl.: C12Q 1/68

(54) **Trex1 as a marker for lupus erythematosus**

(71) Applicant: Max Delbrück Centrum für Molekulare Medizin (MDC) Berlin-Buch;, 13092 Berlin (DE); Technische Universität Dresden, 01069 Dresden (DE)
(72) Inventor: Hübner, Norbert, 13158 Berlin (DE); Lee-Kirsch, Min Ae, 01326 Dresden (DE)
(74) Representative: Krauss, Jan

(57) **Abstract**

The present invention relates a method of diagnosing lupus erythematosus or related autoimmune diseases in a human, said method comprising at least one of detecting a genetic variant, a mutation or polymorphism in the gene encoding for TREX1, detecting a change in the biochemical activity of TREX1, and detecting a change in the expression of the gene encoding for TREX1. The present invention further relates to the use of TREX1 for therapy and as a target for new therapeutical approaches.

## Description

The present invention relates a method of diagnosing lupus erythematosus in a human, said method comprising at least one of detecting a genetic variant (mutation or polymorphism) in the gene encoding for TREX1, detecting a change in the biochemical activity of TREX1, and detecting a change in the expression of the gene encoding for TREX1. The present invention further relates to the use of TREX1 for therapy of lupus erythematosus and related autoimmune diseases and as a target for new therapeutical approaches.

Systemic lupus erythematosus is a complex autoimmune disease with a prevalence of 0.06% in the general population. Its etiology is multifactorial and influenced by both genetic and environmental factors (Alarcon-Riquelme, M. E. (2005) The genetics of systemic lupus erythematosus. J.Autoimmun. 25 Suppl, 46-48; Cunninghame Graham, D. S., Vyse, T. J. (2004) The candidate gene approach: have murine models informed the study of human SLE? Clin Exp.Immunol. 137, 1-7). The clinical picture is characterized by a great variety of symptoms, ranging from mild to lethal. The disease can affect almost any organ in the body, causing arthritis, vasculitis, thrombosis, anemia, heart disease, or kidney failure. Cutaneous findings are a hallmark of the disease and include butterfly rash, discoid lesions, oral ulcers, and alopecia (Sontheimer, R. D. (1997) The lexicon of cutaneous lupus erythematosus--a review and personal perspective on the nomenclature and classification of the cutaneous manifestations of lupus erythematosus. Lupus 6, 84-95). Symptoms usually recur over time, and may also include psychosis and cognitive decline. To date the diagnosis rests on clinical and laboratory criteria defined by the American Rheumatic Association. Thus, the diagnosis is made if 4 of the 11 diagnostic criteria for systemic lupus erythematosus can be found in an affected individual (Mills, J. A. (1994) Systemic lupus erythematosus. N.Engl.J.Med. 330, 1871-1879). However, the insidious onset of the disease and the variable clinical picture at the time of manifestation remain a diagnostic challenge even for the specialist and in many cases the diagnosis is made only when irreparable damage such as renal failure has occurred. The most difficult aspect with respect to management of patients with lupus is to treat the active phase without allowing the treatment to cause long-term damage. Nonsteroidal antiinflammatory drugs remain the mainstay of treatment in patients with polyarthralgias or mild polyarthritis, while for cutaneous manifestations the strict use of sunblock along with judicious use of topical steroids is the treatment of choice. Many patients with systemic or cutaneous lupus erythematosus, however, will need systemic glucocorticoids, antimalarials such as hydroxychloroquine or the more ophthalmotoxic chloroquine, and immunosuppressive agents such as methotrexate, cyclophoaphamide, or azathioprine at some point during the course of the illness.

The formation of immune complexes consisting of autoantibodies against nuclear antigens is thought to be the principal cause of the inflammatory process leading to skins rashes, vasculitis, arthritis, and nephritis (Mills, J. A. (1994) Systemic lupus erythematosus. N.Engl.J.Med. 330, 1871-1879, (Mills, J. A. (1994) Systemic lupus erythematosus. N.Engl.J.Med. 330, 1871-1879; Walport, M. J. (2000) Lupus, DNase and defective disposal of cellular debris. Nat.Genet. 25, 135-136). To date, several susceptibility loci have been identified by both genome-wide and association approaches (Alarcon-Riquelme, M. E. (2005) The genetics of systemic lupus erythematosus. J.Autoimmun. 25 Suppl, 46-48; Cunninghame Graham, D. S., Vyse, T. J. (2004) The candidate gene approach: have murine models informed the study of human SLE? Clin Exp.Immunol. 137, 1-7). However, most of the genetic basis and the molecular pathogenesis of lupus erythematosus remain undefined.

Apart from autosomal recessively inherited deficiencies of complement factors, which play an important role in adaptive immunity, no monogenic form of lupus erythematosus has been identified so far. Thus, selective deficiency of C1q, C1r, C1s, C2, or C4a has been associated with multiple autoimmune diseases including a lupus-like phenotype (Lee, S. L., Wallace, S. L., Barone, R., Blum, L., Chase, P. H. (1978) Familial deficiency of two subunits of the first component of complement. C1r and C1s associated with a lupus erythematosus-like disease. Arthritis Rheum. 21, 958-967; Dragon-Durey, M. A., Quartier, P., Fremeaux-Bacchi, V., Blouin, J., de Barace, C., Prieur, A. M., Weiss, L., Fridman, W. H. (2001) Molecular basis of a selective C1s deficiency associated with early onset multiple autoimmune diseases. J.Immunol. 166, 7612-7616; Hannema, A. J., Kluin-Nelemans, J. C., Hack, C. E., Eerenberg-Belmer, A. J., Mallee, C., van Helden, H. P. (1984) SLE like syndrome and functional deficiency of C1q in members of a large family. Clin Exp.Immunol. 55, 106-114; Provost, T. T., Arnett, F. C., Reichlin, M. (1983) Homozygous C2 deficiency, lupus erythematosus, and anti-Ro (SSA) antibodies. Arthritis Rheum. 26, 1279-1282), while selective deficiency of C3 and C5 leads to high susceptibility to bacterial infections in addition to lupus-like phenotypes (Alper, C. A., Bloch, K. J., Rosen, F. S. (1973) Increased susceptibility to infection in a patient with type II essential hypercatabolism of C3. N.Eng1.J.Med. 288, 601-606; Snyderman, R., Durack, D. T., McCarty, G. A., Ward, F. E., Meadows, L. (1979) Deficiency of the fifth component of complement in human subjects. Clinical, genetic and immunologic studies in a large kindred. Am.J.Med. 67, 638-645).

Genes that have been implicated in the pathogenesis of lupus erythematosus or other autoimmune diseases are for example genes encoding toll like receptor 9, [*TLR9*, MIM *605474] (Ng, M. W., Lau, C. S., Chan, T. M., Wong, W. H., Lau, Y. L. (2005) Polymorphisms of the toll-like receptor 9 (TLR9) gene with systemic lupus erythematosus in Chinese. Rheumatology.(Oxford) 44, 1456-1457; Barrat, F. J., Meeker, T., Gregorio, J., Chan, J. H., Uematsu, S., Akira, S., Chang, B., Duramad, O., Coffman, R. L. (2005) Nucleic acids of mammalian origin can act as endogenous ligands for Toll-like receptors and may promote systemic lupus erythematosus. J.Exp.Med. 202, 1131-1139), deoxyribonuclease I-like 3 [*DNASE1L3*, MIM *602244] (Walport, M. J. (2000) Lupus, DNase and defective disposal of cellular debris. Nat.Genet. 25, 135-136, Wilber, A., O'Connor, T. P., Lu, M. L., Karimi, A., Schneider, M. C. (2003) Dnase113 deficiency in lupus-prone MRL and NZB/W F1 mice. Clin Exp.Immunol. 134, 46-52), ubiquitin-activating enzyme E1-like [*UBE1L*, MIM *191325] (Vinuesa, C. G., Cook, M. C., Angelucci, C., Athanasopoulos, V., Rui, L., Hill, K. M., Yu, D., Domaschenz, H., Whittle, B., Lambe, T., Roberts, I. S., Copley, R. R., Bell, J. I., Cornall, R. J., Goodnow, C. C. (2005) A RING-type ubiquitin ligase family member required to repress follicular helper T cells and autoimmunity. Nature 435, 452-458), protein kinase C delta [*PRKCD*, MIM *176977] (Mecklenbrauker, I., Saijo, K., Zheng, N. Y., Leitges, M., Tarakhovsky, A. (2002) Protein kinase Cdelta controls self-antigen-induced B-cell tolerance. Nature 416, 860-865; Miyamoto, A., Nakayama, K., Imaki, H., Hirose, S., Jiang, Y., Abe, M., Tsukiyama, T., Nagahama, H., Ohno, S., Hatakeyama, S., Nakayama, K. I. (2002) Increased proliferation of B cells and auto-immunity in mice lacking protein kinase Cdelta. Nature 416, 865-869), interleukin 17 receptor B and D [*IL17RB*, MIM *605458; IL17RD, MIM *606807] (Kurasawa, K., Hirose, K., Sano, H., Endo, H., Shinkai, H., Nawata, Y., Takabayashi, K., Iwamoto, I. (2000) Increased interleukin-17 production in patients with systemic sclerosis. Arthritis Rheum. 43, 2455-2463), and the chemokine receptor cluster *[CCR9,* MIM *604738; *CXCR6,* MIM *605163; *XCR1,* MIM *600552; *CCR1,* MIM *601159; *CCR2,* MIM *601267; *CCR3,* MIM *601268; *CCR5,* MIM *601373; *CCRL2,* MIM *608379) (Amoura, Z., Combadiere, C., Faure, S., Parizot, C., Miyara, M., Raphael, D., Ghillani, P., Debre, P., Piette, J. C., Gorochov, G. (2003) Roles of CCR2 and CXCR3 in the T cell-mediated response occurring during lupus flares. Arthritis Rheum. 48, 3487-3496; Eriksson, C., Eneslatt, K., Ivanoff, J., Rantapaa-Dahlqvist, S., Sundqvist, K. G. (2003) Abnormal expression of chemokine receptors on T-cells from patients with systemic lupus erythematosus. Lupus 12, 766-774).

WO 2005/098045 describes a diagnostic method for the detection of SLE in a human body sample through identification of Mycoplasma haemosapiens.

US 6,632,665 describes the isolated nucleic acid molecule encoding the genomic sequence encoding the human 3'-5' exonuclease TREX1. Methods of use include inhibition of exonuclease activity to increase incorporation of nucleotide analogs into DNA in rapidly dividing cells.

WO 2005/051988 broadly describes compositions containing 596 proteins and methods of using those compositions for the diagnosis and treatment of immune related diseases, such as systemic lupus erythematosus. Sequence 200 from WO 2005/051988 encodes for a part of the protein TREX1. WO 2005/051988 focuses on expression analysis, but does not examine mutations or polymorphisms in said genes.

JP 2006-101712 describes a method for diagnosing the systemic lupus erythematosus by analyzing a nucleic acid molecule group containing each of the nucleotide sequences of a plurality of genes selected from the groups of genes specifically expressed in cells presenting in the peripheral blood of a subject having the systemic lupus erythematosus.

Despite the above attempts, there is an ongoing need for the identification of genes responsible for lupus, which may shed light onto the pathogenesis of common forms of connective tissue diseases such as systemic lupus erythematosus, and will allow the development of new diagnostic and therapeutic approaches. Alternatively, also markers for monogenic forms of lupus erythematosus are sought for. In addition, recognition of disease causing genetic variants may also help to define subgroups of patients based on the genetic etiology and may allow prediction of the clinical course and the response to specific therapeutic measures.

In order to solve this problem, according to a first preferred aspect thereof, the present invention provides a method of diagnosing lupus erythematosus in a human, said method comprising at least one of detecting a genetic variant (mutation or polymorphism) in the gene encoding for TREX1, detecting a change in the biochemical activity of TREX1, and detecting a change in the expression of the gene encoding for TREX1. The test can be easily performed at the onset of symptoms even if the clinical picture does not fulfill the diagnostic criteria defined by the American Rheumatic Association. Thus, this test could speed up diagnosis and institution of specific therapy.

Lupus erythematosus, and in particular systemic lupus erythematosus (SLE) is a complex polygenic disease and considered a prototypic autoimmune disease. The locus identified in the present invention does not localize within any hitherto mapped susceptibility locus for systemic lupus erythematosus (Alarcon-Riquelme, M. E. (2005) The genetics of systemic lupus erythematosus. J. Autoimmun. 25 Suppl, 46-48; Cunninghame Graham, D. S., Vyse, T. J. (2004) The candidate gene approach: have murine models informed the study of human SLE? Clin Exp. Immunol. 137, 1-7), previous studies of complex diseases have demonstrated that genome-wide and association approaches may lead to completely different results.

The gene *trex*1 encodes for a DNase III-enzyme and maps to chromosome 3p21.3-p21.2. The protein was falsely used synonymously with ATRIP (ATR-interacting protein), nevertheless, ATRIP-ORF is encoded by 5'prime exons of TREX1 gene in a different reading frame. *Trex*1 includes 11 exons, wherein the coding complete region is localized in exon 11. TREX1 consist of 314 AA, as shown in SEQ ID No. 2. TREX1 appears to be ubiquitously expressed in the human, however some data suggest high expression in lymphocytes. Yet, no role of TREX1 in lupus erythematosus has been described, in contrast, TREX-/- mice die of autoimmune cardiomyocarditis (Morita et al. (2004) Gene-targeted mice lacking the Trex1 (DNase III) 3'-5'DNA exonuclease develop inflammatory myocarditis. Mol. Cell. Biol. 24, 6719-6727).

In yet another embodiment, the present invention concerns a method of diagnosing lupus erythematosus in a human, wherein said diagnosis further includes the step of an analysis of the risk to develop a lupus erythematosus or an analysis of the risk for a relapse of lupus erythematosus in said human.

In yet another embodiment, the present invention concerns a method of diagnosing lupus erythematosus in a human according to the present invention, wherein detection of said change of expression comprises a quantitative or qualitative detection method. Such methods are well known and comprise methods that quantify the amount of TREX1-protein or of TREX1-encoding mRNA. Preferred is a method of diagnosing lupus erythematosus in a human according to the present invention, wherein detection of said change of expression comprises using a TREX1-specific antibody or a trex1-mRNA specific RT-PCR. Thus, the detection may be qualitative or quantitative, and may be performed in comparison with monitoring a complex formation in a control sample of known normal tissue cells of the same cell type. A larger quantity of complexes formed in the test sample indicates the presence or absence of lupus erythematosus in the human from which the test tissue cells were obtained. The TREX1-specifc antibody preferably carries a detectable label. Complex formation can be monitored, for example, by light microscopy, flow cytometry, fluorimetry, or other techniques known in the art. The test sample is usually obtained from an individual suspected of having lupus erythematosus.

In another embodiment, the invention provides a method for determining the presence of TREX1 polypeptide in a sample obtained from an individual suspected of having lupus erythematosus comprising exposing a test sample of cells suspected of containing the TREX1-polypeptide to an anti-TREX1 antibody, and determining the binding of said antibody to said cell sample. In a specific aspect, the sample comprises a cell suspected of containing the TREX1-polypeptide and the antibody binds to the cell. The antibody is preferably detectably labeled and/or bound to a solid support. The present invention further provides said specific anti-TREX1 antibodies. Exemplary antibodies include polyclonal, monoclonal, humanized, bispecific, and heteroconjugate antibodies.

In another embodiment, the invention provides a method of diagnosing SLE in a mammal which comprises detecting the presence or absence of the TREX1 polypeptide in a test sample of tissue cells obtained from said mammal, wherein the presence or absence of the TREX1 polypeptide in said test sample is indicative of the presence of SLE in said mammal.

Most preferred is the use of a TREX1-specific antibodies in order to distinguish between mutated and non-mutated TREX1-polypeptides, as described herein in Table 1 below, or in order to detect quantitative tissues-specific changes in protein expression.

In yet another embodiment, the present invention concerns a method of diagnosing lupus erythematosus in a human according to the present invention, wherein detection of said change of biochemical activity comprises a 3' nucleotide excision assay. Examples of such assays are known in the literature and described, for example, in Mazur et al. (Mazur DJ, Perrino FW. Excision of 3' termini by the Trex1 and TREX2 3'-->5' exonucleases. Characterization of the recombinant proteins. J Biol Chem. 2001 May 18;276(20):17022-9. Epub 2001 Mar 6.).

A particularly preferred embodiment of the present invention then relates to a method of diagnosing lupus erythematosus in a human according to the present invention, wherein said method comprises the steps of: a) obtaining a biological test sample from a human to be diagnosed, preferably a human suspected of having or being predisposed to lupus erythematosus, b) analyzing the sequence of the gene encoding for TREX1 in said sample, c) comparing said sequence as analyzed with a sequence of the gene encoding for TREX1 in a control sample from a healthy individual, and d) diagnosing lupus erythematosus in said human to be diagnosed based on the differences between the two samples, wherein the presence of a genetic variant (mutation or polymorphism) in the test sample as compared to the control sample is indicative of the risk and/or presence of lupus erythematosus.

For example, antibodies, including antibody fragments, can be used to qualitatively or quantitatively detect the expression of TREX1 protein ("marker gene product"). The antibody preferably is equipped with a detectable, e.g., fluorescent label, and binding can be monitored by light microscopy, flow cytometry, fluorimetry, or other techniques known in the art. It is speculated by the inventors that the presence of a mutation or mutations in the gene encoding for TREX1 will have an effect in that the activity and/or presence of TREX1 is reduced. Since TREX1 is regarded as playing an important role in the degradation/control of single-stranded DNA in the nucleus of the cell, it is likely that a malfunction of TREX1 will lead to an increased level of single-stranded DNA which constitutes an (auto)immune target and might be one factor for the development of the autoimmune diseases as described herein.

In situ detection of antibody binding to the TREX1 gene product can be performed, for example, by immunofluorescence or immunoelectron microscopy. For this purpose, a histological specimen is removed from the patient, and a labeled antibody is applied to it, preferably by overlaying the antibody on a biological sample. This procedure also allows for determining the distribution of the marker gene product in the tissue examined. It will be apparent for those skilled in the art that a wide variety of histological methods are readily available for in situ detection.

Preferred is a method of diagnosing lupus erythematosus in a human according to the present invention, wherein analyzing the sequence of the gene encoding for TREX1 comprises the detection of genetic variants (mutations and polymorphisms) in the regulatory regions, introns and/or exons, in particular within the coding exon of the *trex1*-gene. Thus, the term "gene encoding for TREX1" in the context of the present invention encompasses the coding as well as the non-coding regions of the gene, together with any regulatory regions that are responsible for a control of TREX1-expression, such as promoter and enhancer regions, as well as 5'- and/or 3'-non-coding regions of *trexl.* Most preferred is an analysis of the region as depicted in SEQ ID No. 1 or 3.

Mutations are differences in the nucleic acid sequence of a gene that change the encoded protein or its expression thereby changing its function and/ or its transcriptional regulation.

Polymorphisms are differences in the nucleic acid sequence of a gene and are differences in DNA sequence among individuals. Genetic variations occurring in more than 1% of a population would be considered useful polymorphisms for the present analysis. Therefore, all methods for analyzing nucleic acid sequences can be used in the context of the present invention. Preferred is a method of diagnosing lupus erythematosus in a human according to the present invention, wherein detection of said genetic variants (mutations or polymorphisms) comprises at least one of an analysis comprising hybridization, restriction enzyme analysis, and sequencing.

A particularly preferred embodiment of the present invention then relates to a method of diagnosing lupus erythematosus in a human according to the present invention, wherein said genetic variants (mutations and polymorphisms) to be detected are selected from the group of genetic variants as listed in Table 1.

Table 1 below shows the results of the sequence analysis of all exons including flanking intronic regions of the Trex1 gene among 90 unrelated patients with SLE and 96 unrelated healthy controls. Several genetic variants that were present only among individuals affected with SLE and not among healthy controls could be identified.

| **Position and name of polymorphism according to genomic sequence (SEQ ID No. 1)** | **Number of alleles found in SLE cases** **(n = 90)** | **Number of alleles found in controls** **(n = 96)** | **Description** |
|---|---|---|---|
| g.3829A>T | 1 (T-allele) | 0 | Synonymous¹ |
| g.3858A>G | 3 (G-allele) | 5 | Synonymous¹ |
| g.4032A>C rs11925638 | 1 (C-allele) | 0 | Synonymous¹ |
| g.11170C>T rs35240314 | 1 (T-allele) | 0 | Synonymous¹ |
| g.14283G>A | 1 (A-allele) | 0 | Synonymous¹ |
| g.14384C>T | 1 (T-allele) | 0 | Synonymous¹ |
| g.14455T>C | 1 (C-allele) | 0 | Synonymous¹ |
| g.17696C>T rs34426134 | 1 (T-allele) | 0 | Synonymous¹ |
| g.18682C>A rs3135936 | 2 (A-allele) | 0 | Synonymous¹ |
| g.18743G>A | 2 (A-allele) | 4 | Synonymous¹ |
| g.18853A>T | 1 (T-allele) | 0 | Synonymous¹ |
| g.20131 T>C rs3135941 | 42 (T-allele) | 26 | Synonymous¹ |
| g.20570G>A | 1 (A-allele) | 0 | D18N² |
| g.20852C>A | 1 (A-allele) | 1 | L112M |
| g.21049C>T rs11797 | 73 (T-allele) | 69 | Synonymous¹ |
| g.21197G>A | 1 (A-allele) | 0 | G227S |
| g.21238G>C | 1 (C-allele) | 0 | R240S |
| g.21257G>C | 1 (C-allele( | 0 | A247P |
| g.21315A>G | 1 (G-allele) | 0 | E266G |
| g.21330^ 21331insAA | 1 (insAA) | 0 | K271fs272R |
| g.21430G>A rs3135945 | 4 (A-allele) | 0 | Synonymous¹ |
| g.21500T>C rs3135946 | 3 (C-allele) | 0 | 3'-untranslated region |

| | | | |
|---|---|---|---|
| ¹: with reference to Trex1 open reading frame. ²: mutation in family with monogenic chilblain lupus. | | | |

Lupus erythematosus usually is a multifactorial disease, i.e. several genes are involved in the development and/or the predisposition of said disease. The present invention now provides a novel marker for lupus erythematosus, the gene for TREX1, which can either be used as an additional marker in combination with existing lupus-markers (see above), or even as a mono-marker for the presence of lupus erythematosus. Another aspect of the present invention therefore relates to a method of diagnosing lupus erythematosus in a human according to the present invention, wherein said lupus erythematosus is selected from systemic lupus erythematosus, subacute cutaneous lupus erythematosus, cutaneous lupus erythematosus including chilblain lupus erythematosus, rheumatoid arthritis, Sjogren's syndrome, autoimmune nephritis, autoimmune vasculitis, autoimmune hepatitis, autoimmune carditis, autoimmune encephalitis, autoimmune mediated hematological disease, and other autoimmune diseases.

A particularly preferred embodiment of the present invention then relates to a method of diagnosing lupus erythematosus in a human according to the present invention, which further comprising the analysis of other genetic markers for lupus erythematosus, such as MICA, CTLA4, CD154 (3'UTR), PDCD1, RNUX1, PTP22, SCL22A4, SUMO4, and IRF5). Additional markers are known from the literature (see, for example, Roberton CA, Vyse TJ. The genetics of systemic lupus erythematosus. Exp Nephrol. 2000 Jul-Oct;8(4-5):194-202.; Gambelunghe G, Gerli R, Bocci EB, Del Sindaco P, Ghaderi M, Sanjeevi CB, Bistoni O, Bini V, Falorni A. Contribution of MHC class I chain-related A (MICA) gene polymorphism to genetic susceptibility for systemic lupus erythematosus. Rheumatology (Oxford). 2005 Mar;44(3):287-92. Epub 2004 Nov 2. Torres B, Aguilar F, Franco E, Sanchez E, Sanchez-Roman J, Jimenez Alonso J, Nunez-Roldan A, Martin J, Gonzalez-Escribano MF. Association of the CT60 marker of the CTLA4 gene with systemic lupus erythematosus. Arthritis Rheum. 2004 Jul;50(7):2211-5.; Citores MJ, Rua-Figueroa I, Rodriguez-Gallego C, Durantez A, Garcia-Laorden MI, Rodriguez-Lozano C, Rodriguez-Perez JC, Vargas JA, Perez-Aciego P. The dinucleotide repeat polymorphism in the 3'UTR of the CD154 gene has a functional role on protein expression and is associated with systemic lupus erythematosus. Ann Rheum Dis. 2004 Mar;63(3):310-7.; Tokuhiro S, Yamada R, Chang X, Suzuki A, Kochi Y, Sawada T, Suzuki M, Nagasaki M, Ohtsuki M, Ono M, Furukawa H, Nagashima M, Yoshino S, Mabuchi A, Sekine A, Saito S, Takahashi A, Tsunoda T, Nakamura Y, Yamamoto K. An intronic SNP in a RUNX1 binding site of SLC22A4, encoding an organic cation transporter, is associated with rheumatoid arthritis.Nat Genet. 2003 Dec;35(4):341-8. Epub 2003 Nov 9., Reddy MV, Johansson M, Sturfelt G, Jonsen A, Gunnarsson I, Svenungsson E, Rantapaa-Dahlqvist S, Alarcon-Riquelme ME. The R620W C/T polymorphism of the gene PTPN22 is associated with SLE independently of the association of PDCD1. Genes Immun. 2005 Dec;6(8):658-62, Prokunina L, Castillejo-Lopez C, Oberg F, Gunnarsson I, Berg L, Magnusson V, Brookes AJ, Tentler D, Kristjansdottir H, Grondal G, Bolstad AI, Svenungsson E, Lundberg I, Sturfelt G, Jonssen A, Truedsson L, Lima G, Alcocer-Varela J, Jonsson R, Gyllensten UB, Harley JB, Alarcon-Segovia D, Steinsson K, Alarcon-Riquelme ME. A regulatory polymorphism in PDCD1 is associated with susceptibility to systemic lupus erythematosus in humans. Nat Genet. 2002 Dec;32(4):666-9. Epub 2002 Oct 28. Orozco G, Sanchez E, Gomez LM, Gonzalez-Gay MA, Lopez-Nevot MA, Torres B, Ortego-Centeno N, Jimenez-Alonso J, de Ramon E, Sanchez Roman J, Anaya JM, Sturfelt G, Gunnarsson I, Svennungsson E, Alarcon-Riquelme M, Gonzalez-Escribano MF, Martin J. Study of the role of functional variants of SLC22A4, RUNX1 and SUMO4 in systemic lupus erythematosus. Ann Rheum Dis. 2006 Jun;65(6):791-5. Epub 2005 Oct 25., Graham RR, Kozyrev SV, Baechler EC, Reddy MV, Plenge RM, Bauer JW, Ortmann WA, Koeuth T, Gonzalez Escribano MF; Argentine and Spanish Collaborative Groups; Pons-Estel B, Petri M, Daly M, Gregersen PK, Martin J, Altshuler D, Behrens TW, Alarcon-Riquelme ME. A common haplotype of interferon regulatory factor 5 (IRF5) regulates splicing and expression and is associated with increased risk of systemic lupus erythematosus. Nat Genet. 2006 May;38(5):550-5. Epub 2006 Apr 16).

The marker of the present invention can be used by the attending physician in order to apply a preventive treatment to a human in order to ameliorate a relapse or even avoid onset of the autoimmune disease (as discussed in, for example, Harel M, Shoenfeld Y. Predicting and preventing autoimmunity, myth or reality? Ann N Y Acad Sci. 2006 Jun;1069:322-45.). Another aspect of the method of diagnosing lupus erythematosus in a human according to the present invention, therefore further includes the step of selecting measures for secondary or primary prevention based on said diagnosis. Such measures could be the prevention of antibodies that are accrued by patients throughout a foreseen course during the years prior to the clinical symptoms. Another aspect of the present invention relates to a classification of the lupus erythematosus in order to provide a more effective therapy based on a precise diagnosis of the pathological condition and the individual needs of the patient ("personalized treatment"), which can be readily performed by the attending physician based on the information as provided herein as well as the respective literature (see, for example, Centola M, Frank MB, Bolstad AI, Alex P, Szanto A, Zeher M, Hjelmervik TO, Jonsson R, Nakken B, Szegedi G, Szodoray P. Genome-scale assessment of molecular pathology in systemic autoimmune diseases using microarray technology: a potential breakthrough diagnostic and individualized therapy-design tool. Scand J Immunol. 2006 Sep;64(3):236-42.; and references as cited therein).

A particularly preferred embodiment of the present invention then relates to a method for identifying a compound which modifies the activity of TREX1, said method comprising contacting cells expressing TREX1 with a candidate compound, and identifying said compound by determining the activity of TREX1 in response to said candidate compound. Preferred is a method of identifying a compound which modifies the activity of TREX1 according to the present invention, wherein the activity of TREX1 is selected from the biochemical activity or the expression of the gene encoding for TREX1. The compound can be an agonist or antagonist of the TREX1 activity.

The term "antagonist" is used in the broadest sense, and includes any molecule that partially or fully blocks, inhibits, or neutralizes the biological activity of a native TREX1 polypeptide disclosed herein. In a similar manner, the term "agonist" is used in the broadest sense and includes any molecule that mimics a biological activity of a native TREX1 polypeptide disclosed herein. Suitable agonist or antagonist molecules specifically include agonist or antagonist antibodies or antibody fragments, fragments or amino acid sequence variants of native TREX1 polypeptides, peptides, antisense oligonucleotides, small organic molecules, etc. Methods for identifying agonists or antagonists of a TREX1 polypeptide may comprise contacting a TREX1 polypeptide with a candidate agonist or antagonist molecule and measuring a detectable change in one or more biological activities normally associated with the TREX1 polypeptide, such as described herein.

Preferred is a method for identifying a compound that modifies the activity of TREX1 according to the present invention, wherein said determining of said change of expression comprises a TREX1 antibody and/or an RT-PCR and/or a 3' nucleotide excision assay, as described above for the diagnosis. Further preferred is a method for identifying a compound that modifies the activity of TREX1 according to the present invention, wherein said candidate compound is a TREX1 antisense nucleic acid or a TREX1-derived polypeptide, which is optionally mutated and/or chemically modified. Preferred is a TREX1-peptide that is mutated in order to function as a competitor for the TREX1 biological function in the cell.

In yet another embodiment, the present invention concerns a diagnostic kit for lupus erythematosus, comprising: a container comprising materials for performing a diagnostic method according to the present invention, together with suitable carriers and instructions for use.

In another embodiment, the present invention concerns an lupus erythematosus-related disease diagnostic kit, comprising an anti-TREX1 antibody and a carrier in suitable packaging. The kit preferably contains instructions for using the antibody to detect the presence of the TREX1 polypeptide, in particular mutated TREX1 polypeptide as described herein. Preferably, the carrier is pharmaceutically acceptable.

In another embodiment of the invention, a kit useful for the diagnosis or treatment of the disorders described herein is provided. The kit comprises a container and an instruction. Suitable containers include, for example, bottles, vials, syringes, and test tubes. The containers may be formed from a variety of materials such as glass or plastic. The container holds a composition which is effective for diagnosing or treating lupus erythematosus, and may have a sterile access port (for example the container may be an intravenous solution bag or a vial having a stopper pierceable by a hypodermic injection needle). The active agent in the composition is usually a polypeptide, a compound or an antibody of the invention. An instruction or label on, or associated with, the container indicates that the composition is used for diagnosing or treating the lupus-condition of choice. The kit may further comprise a second container comprising a pharmaceutically-acceptable buffer, such as phosphate-buffered saline, Ringer's solution and dextrose solution. It may further include other materials desirable from a commercial and user standpoint, including other buffers, diluents, filters, needles, syringes, and package inserts with instructions for use.

Compounds identified by the above described screening assays can be formulated into a pharmaceutical composition, using standard techniques well known in the art.

In another embodiment, the invention provides a pharmaceutical preparation, comprising a compound as identified according to the present invention, together with a pharmaceutically acceptable carrier and/or excipient.

In another embodiment, the invention relates to the use of a compound as identified according to the present invention, or a pharmaceutical preparation according to the present invention for the production of a medicament for the treatment of systemic lupus erythematosus, subacute cutaneous lupus erythematosus, cutaneous lupus erythematosus including chilblain lupus erythematosus, rheumatoid arthritis, Sjogren's syndrome, autoimmune nephritis, autoimmune vasculitis, autoimmune hepatitis, autoimmune carditis, autoimmune encephalitis, autoimmune mediated hematological disease, and other autoimmune diseases.

Therapeutic formulations of the active compound, preferably comprising a polypeptide, antibody or compound as screened of the invention, are prepared for storage by mixing the active molecule having the desired degree of purity with optional pharmaceutically acceptable carriers, excipients or stabilizers(Remington's Pharmaceutical Sciences 16th edition, Osol, A. Ed. [1980]), e.g. in the form of lyophilized formulations or aqueous solutions.

Acceptable carriers, excipients, or stabilizers are nontoxic to recipients at the dosages and concentrations employed, and include buffers such as phosphate, citrate, and other organic acids; antioxidants including ascorbic acid and methionine ; preservatives (such as octadecyl-dimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins ; hydrophilic polymers such as polyvinylpyrrolidone ; amino acids such as glycine, glutamin, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g., Zn-protein complexes); and/or non-ionic surfactants such as TWEEN^{™}, PLURONICS^{™} or polyethylene glycol (PEG).

The compositions useful in the treatment of lupus include, without limitation, proteins, antibodies, small organic molecules, peptides, phosphopeptides, antisense and ribozyme molecules, triple helix molecules, etc. that inhibit or stimulate the function of TREX1. For example, antisense RNA and RNA molecules act to directly block the translation of mRNA by hybridizing to targeted TREX1-mRNA and preventing protein translation. When antisense DNA is used, oligodeoxyribonucleotides derived from the translation initiation site, e.g., between about-10 and +10 positions of the target gene nucleotide sequence, are preferred. Ribozymes are enzymatic RNA molecules capable of catalyzing the specific cleavage of RNA. Ribozymes act by sequence-specific hybridization to the complementary target RNA, followed by endonucleolytic cleavage. Specific ribozyme cleavage sites within a potential RNA target can be identified by known techniques. For further details see, e. g., Rossi, Current Biology 4, 469-471 (1994), and PCT publication No. WO 97/33551 (published September 18,1997).

Nucleic acid molecules in triple helix formation used to inhibit transcription should be single-stranded and composed of deoxynucleotides. The base composition of these oligonucleotides is designed such that it promotes triple helix formation via Hoogsteen base pairing rules, which generally require sizeable stretches of purines or pyrimidines on one strand of a duplex. For further details see, e. g., PCT publication No. WO 97/33551, supra.

These molecules can be identified by any or any combination of the screening assays discussed above and/or by any other screening techniques well known for those skilled in the art.

Lipofections or liposomes can also be used to deliver the TREX1-related molecules into cells. Where antibody fragments are used, the smallest inhibitory fragment which specifically binds to the binding domain of the target protein TREX1 is preferred. For example, based upon the variable region sequences of an antibody, peptide molecules can be designed which retain the ability to bind the target protein sequence. Such peptides can be synthesized chemically and/or produced by recombinant DNA technology (see, e.g., Marascoet al., Proc. Natl. Acad. Sci. USA 90 7889-7893[1993]).

The formulation herein may also contain more than one active compound as necessary for the particular lupus indication being treated, preferably those with complementary activities that do not adversely affect each other. Alternatively, or in addition, the composition may comprise a cytotoxic agent, cytokine or growth inhibitory agent. Such molecules are suitably present in combination in amounts that are effective for the purpose intended.

The active TREX1-related molecules may also be entrapped in microcapsules prepared, for example, by coacervation techniques or by interfacial polymerization, for example, hydroxymethylcellulose or gelatin- microcapsules and poly- (methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

The formulations to be used for in vivo administration must be sterile. This is readily accomplished by filtration through sterile filtration membranes. Sustained-release preparations of the TREX1-related molecules may be prepared. Suitable examples of sustained-release preparations include semipermeable matrices of solid hydrophobic polymers containing the antibody, which matrices are in the form of shaped articles, e.g., films, or microcapsules. Examples of sustained-release matrices include polyesters, hydrogels (for example, poly (2-hydroxyethyl-methacrylate), or poly(vinylalcohol)), polylactides (U. S. Pat. No. 3,773, 919), copolymers of L-glutamic acid and y-ethyl-L-glutamate, non-degradable ethylene-vinyl acetate, degradable lactic acid-glycolic acid copolymers such as the LUPRON DEPOT (injectable microspheres composed of lactic acid-glycolic acid copolymer and leuprolide acetate), and poly-D- (-)-3-hydroxybutyric acid. While polymers such as ethylene-vinyl acetate and lactic acid-glycolic acid enable release of molecules for over 100 days, certain hydrogels release proteins for shorter time periods. When encapsulated antibodies remain in the body for a long time, they may denature or aggregate as a result of exposure to moisture at 37°C, resulting in a loss of biological activity and possible changes in immunogenicity. Rational strategies can be devised for stabilization depending on the mechanism involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, and developing specific polymer matrix compositions.

In yet another embodiment, the present invention concerns a method of treating lupus erythematosus in a human in need thereof, comprising administering to said human a therapeutically effective amount of a compound as identified according to the present invention, or a pharmaceutical preparation according to the present invention. In yet another embodiment, the present invention concerns a method for treating SLE in a human that suffers therefrom comprising administering to the mammal a TREX-1 antisense-nucleic acid molecule, a agonist of a TREX1 polypeptide or an antagonist of a TREX1 polypeptide, wherein said agonist or antagonist preferably is an anti-TREX1 antibody. In another preferred embodiment, the nucleic acid is administered via ex vivo gene therapy. In a further preferred embodiment, the nucleic acid is comprised within a vector, more preferably an adenoviral, adeno-associated viral, lentiviral or retroviral vector.

"Treatment" refers to both therapeutic treatment and prophylactic or preventative measures, wherein the object is to prevent or slow down (lessen) the targeted pathologic condition or disorder, in particular lupus erythematosus. Those in need of treatment include those already with the disorder as well as those prone to have the disorder or those in whom the disorder is to be prevented.

Preferred is a method of treating lupus erythematosus in a human in need thereof according to the present invention, wherein a second immunosuppressive agent is administered. The formulation herein may also contain more than one active compound as necessary for the particular lupus indication being treated, preferably those with complementary activities that do not adversely affect each other. Alternatively, or in addition, the composition may comprise a cytotoxic agent, cytokine or growth inhibitory agent. Such molecules are suitably present in combination in amounts that are effective for the purpose intended. Administration "in combination with" one or more further therapeutic agents includes simultaneous (concurrent) and consecutive administration in any order.

Most preferred is a method of treating lupus erythematosus in a human in need thereof according to the present invention, wherein said lupus erythematosus is selected from systemic lupus erythematosis, subacute cutaneous lupus erythematosus, cutaneous lupus erythematosus including chilblain lupus erythematosus, rheumatoid arthritis, Sjogren's syndrome, autoimmune nephritis, autoimmune vasculitis, autoimmune hepatitis, autoimmune carditis, autoimmune encephalitis, autoimmune mediated hematological disease, and other autoimmune diseases.

SLE is an autoimmune inflammatory connective tissue disease with variable symptomotolgy including fever, joint pains or arthritis resembling rheumatoid arthritis, erythematous skin lesions and internal organ disease that may lead to cardiac or renal failure. For the past 30 years, cyclophosphamide has been used to treat SLE. Cyclophosphamide however, generally inhibits inflammation and has many side effects. Improvements in the understanding of the molecular events involved in the progression of SLE have lead to the trial of new therapeutic agents with less side effects and more efficacy due to the specific targeting of identified components of SLE. Therapeutics that are immunosuppressive are Mycophenolatemofetil (MMF), Leflunomide, and Tacrolimus (FI506). These molecules have a suppressive effect on the immune system, and in limited trials, have had fewer and less severe side effects than cyclophosphamide.

Biological therapies such as Rituxan', anti-C5b, anti-CD40L, anti-BLYS and anti-IL10, are all antibody therapies that have few side effects and target one specific molecule or pathway that is believed to be significant in the progression of SLE. Rituxan, for example, has been used to deplete B cells in SLE patients, and this correlated with improvements in arthritis, rash and fatigue. The biological therapies also have the advantage of being well tolerated, and can be used in combination with established agents such as cyclophosphamide. In the most severe cases of SLE, hematopoeitic stem cell replacement has caused remission in SLE patients, but the long-term results of these cell transplantations has not yet been determined. While cyclophosphamide has remained the major drug treatment of choice for SLE, the new therapies discussed herein that target TREX1 show great promise. The wider range of treatments now in development may mean improved control of this disease.

The compounds of the present invention, e. g., polypeptides, compounds or antibodies, are administered to a human, in accord with known methods, such as intravenous administration as a bolus or by continuous infusion over a period of time, by intramuscular, intraperitoneal, intracerobrospinal, subcutaneous, intra-articular, intrasynovial, intrathecal, oral, topical, or inhalation (intranasal, intrapulmonary) routes. Intravenous or inhaled administration of polypeptides, compounds and antibodies is preferred.

"Carriers" as used herein include pharmaceutically acceptable carriers, excipients, or stabilizers which are nontoxic to the cell or mammal being exposed thereto at the dosages and concentrations employed. Often the physiologically acceptable carrier is an aqueous pH buffered solution. Examples of physiologically acceptable carriers include buffers such as phosphate, citrate, and other organic acids; antioxidants including ascorbic acid; low molecular weight (less than about 10 residues) polypeptide; proteins, such as serum albumin, gelatin, or immunoglobulins; hydrophilic polymers such as polyvinyl pyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN^{™}, polyethylene glycol (PEG), and PLURONICS^{™}.

The following examples are offered for illustrative purposes only, and are not intended to limit the scope of the present invention in any way.

All patent and literature references cited in the present specification are hereby incorporated by reference in their entirety.
SEQ ID No. 1 shows the genomic nucleic acid sequence of *trex1.*
SEQ ID No. 2 shows the amino acid sequence of TREX1.
SEQ ID No. 3 shows the nucleic acid sequence of *trex1-cDNA.*

### EXAMPLES

TREX1 was identified in a German family showing clinical phenotypes that were consistent with the diagnosis of chilblain lupus, a rare cutaneous form of lupus erythematosus (Hutchinson J (1888) Harveian lectures on lupus. On the various forms of lupus vulgaris and erythematosus. British Medical Journal 1, 118). The presence of histological and serological findings typically seen in lupus constitute diagnostic criteria and differentiates chilblain lupus from true cold induced pemiosis or chilblains (Franceschini, F., Calzavara-Pinton, P., Quinzanini, M., Cavazzana, I., Bettoni, L., Zane, C., Facchetti, F., Airo, P., McCauliffe, D. P., Cattaneo, R. (1999) Chilblain lupus erythematosus is associated with antibodies to SSAIRo. Lupus 8,215-219). It occurs predominantly in adult women and has only rarely been described in children (Millard, L. G., Rowell, N. R. (1978) Chilblain lupus erythematosus (Hutchinson). A clinical and laboratory study of 17 patients. Br.J.Dermatol. 98, 497-506; Weston, W. L., Morelli, J. G. (2000) Childhood pemio and cryoproteins. Pediatr.Dermatol. 17,97-99). Apart from two affected brothers, no familial occurrence has been described to date (Weston, W. L., Morelli, J. G. (2000) Childhood pemio and cryoproteins. Pediatr.Dermato1. 17,97-99). Progression to systemic lupus erythematosus has been reported in up to 18% and is thus, comparable to the progression rate of classic chronic cutaneous lupus erythematosus (Millard, L. G., Rowell, N. R. (1978) Chilblain lupus erythematosus (Hutchinson). A clinical and laboratory study of 17 patients. Br.J.Dermato1. 98, 497-506).

The vertical transmission of the disease in both males and females and the consistency of the phenotype suggested autosomal dominant inheritance with high penetrance. The phenotype of all family members was established through direct clinical exam or review of medical records. All participating individuals granted informed written consent prior to the study. In order to map the disease locus, whole-genome linkage analysis was carried out on genomic DNA from peripheral blood leukocytes from 26 family members including 14 affected individuals using the Affymetrix GeneChip Human Mapping 10K Xba 142 2.0 Array. Genotyping of microsatellite markers was carried out with fluorescently labeled primers on an ABI3100 Sequencer (Applied Biosystems). GRR and PedCheck were used to verify relationship and gender and to identify Mendelian errors (Abecasis, G. R., Chemy, S. S., Cookson, W. O., Cardon, L. R. (2001) GRR: graphical representation of relationship errors. Bioinformatics. 17, 742-743; O'Connell, J. R., Weeks, D. E. (1998) PedCheck: a program for identification of genotype incompatibilities in linkage analysis. Am.J.Hum.Genet. 63,259-266). Non-parametric and parametric linkage analysis was carried out with Merlin 1.0 (Abecasis, G. R., Cherny, S. S., Cookson, W. O., Cardon, L. R. (2002) Merlin-rapid analysis of dense genetic maps using sparse gene flow trees. Nat.Genet. 30,97-101). For parametric analysis a fully penetrant autosomal dominant trait without phenocopy and a disease allele frequency of 0.001 were assumed.

The disease locus was localized on chromosome 3p21-3p14 with a maximum LOD score of 5.04 (Figure 4). Non-parametric analysis confirmed this locus with a Z-mean score of 6.2. A common haplotype, that was shared by all affected and absent in all unaffected individuals, was identified. Based on critical recombination events in 2 affected individuals the disease interval was defined to a 13.8 cM region delimited by markers rs704920 and d3s1300 (Figure 4). The mapped interval comprises 16.4 Mb and includes over 100 known genes (Ensembl v38).

By systematic mining of the Ensembl and GeneCards databases the function and expression of all genes were investigated and candidate genes selected for sequencing. All coding exons including flanking intronic regions were sequenced on an ABI3100 sequencer according to standard procedures. Priority was given to genes that had been implicated in the pathogenesis of lupus erythematosus or other autoimmune diseases such as the genes encoding toll like receptor 9, [*TLR9*, MIM *605474] deoxyribonuclease I-like 3 [*DNASE1L3*, MIM *602244], ubiquitin-activating enzyme E1-like [*UBE1L,* MIM *191325], protein kinase C delta [*PRKCD,* MIM *176977], interleukin 17 receptor B and D [*IL17RB,* MIM *605458; IL17RD, MIM *606807], and the chemokine receptor cluster *[CCR9,* MIM *604738; *CXCR6*, MIM *605163; *XCR1,* MIM *600552; *CCR1,* MIM *601159; *CCR2,* MIM *601267; *CCR3,* MIM *601268; *CCR5,* MIM *601373; *CCRL2,* MIM *608379). The inventors also investigated the collagen VII alpha-1 gene [*COL7A1*, MIM *120120], since it is highly expressed in the skin and known to cause autosomal dominant bullous epidermolysis dystrophica [MIM *131750], although blistering is not a feature of chilblain lupus. Several known and novel SNPs were identified, but no pathogenic mutation was observed within the regions analyzed. All novel coding SNPs were interrogated for segregation with the trait and their allele frequencies estimated by genotyping 100 unrelated healthy controls (data not shown).

Comparative sequencing of the Trex1 gene located within the mapped disease-haplotype revealed a heterozygous change at position 20570 of the genomic sequence (g.20570G>A), that leads to the exchange of an aspartic acid with an asparagine residue (D18N). This mutation was present in all family members affected with chilblain lupus and absent in all non-affected members. The mutation lies within the first of three EXO domains, EXO I, of the Trex1 gene, which generate the exonuclease active site of the Trex1 enzyme. The highly conserved EXO motifs are characteristic features of a number of exonucleases from different species including the proofreading exonucleases of bacterial replicative DNA polymerases (Mazur et al. Identification and expression of the Trex1 and Trex2 cDNA sequences encoding mammalian 3'-5' exonucleases, J. Biol. Chem. 1999, 274, 19655-19660, Höss et al. A human DNA editing enzyme homologue to the Escherichia coli DnaQ/MutD protein, EMBO 1999, 18, 3868-3875). Within these EXO domains, 4 amino acid residues, an aspartic acid and a glutamic acid contributed by EXO I and 2 aspartic acid residues contributed each by the EXO II and EXO III domain, respectively, are critical for metal ion binding and show the highest degree of conservation. The mutation D18N affects the first highly conserved aspartic acid within the EXO I motif and is, thus, predicted to impede enzymatic activity. Analysis of enzymatic activity of the Trex1 mutant was performed in nuclear extracts of Epstein Barr-virus-transformed lympholastoid cells from 3 affected individuals as described by Morita et al. (Morita et al. (2004) Gene-targeted mice lacking the Trex1 (DNase III) 3'-5'DNA exonuclease develop inflammatory myocarditis. Mol. Cell. Biol. 24, 6719-6727). Enzyme assays were performed by incubation of purified nuclear extracts with poly(dA) substrate, which was 3'-end labeled by the incorporation of [³²P]dAMP by terminal transferase. The substrate was ethanol precipitated in the presence of denatured calf thymus carrier DNA and the radioactive material released into the supernatant determined by scintillation counting. Enzymatic activity was found to be reduced to up to 50% in affected individuals as compared to unrelated healthy control samples suggesting that the mechanism underlying the phenotype may be mediated by haploinsufficiency of the Trex1 gene.

Because of the phenotypic overlap of chilblain lupus with common forms of lupus erythematosus we tested the hypothesis that patients with sporadic forms of systemic lupus erythematosus carry mutations within the Trex1 gene. Using a case-control approach a cohort of 90 unrelated patients with systemic lupus erythematosus diagnosed according to the criteria of the American Rheumatic Association was compared with 96 unrelated healthy individuals by direct sequencing of all exons including flanking intronic regions of the Trex1 gene. Among patients with Lupus erythematodus 6 mutations, 5 missense and 1 frrame shift mutation, were identified, none of which was found in healthy controls (Table 1). In addition, several genetic variants were found, both within non-coding exonic and intronic regions. Of those 11 were were present only among patients with SLE and not in healthy controls, and 4 were found to occur at different frequencies (Table 1). These data show, that genetic variants of the Trex1 gene occur more frequently among patient with lupus erythematosus suggesting that changes in the protein and/ or the regulation of its transcription may play a causal role in the pathogenesis of lupus erythematosus.

## Claims

1. A method of diagnosing lupus erythematosus and related autoimmune diseases in a human, said method comprising at least one of detecting a genetic variant (mutation or polymorphism) in the gene encoding for TREX1, detecting a change in the biochemical activity of TREX1, and detecting a change in the expression of the gene encoding for TREX1.

2. The method of diagnosing lupus erythematosus in a human according to claim 1, wherein said diagnosis further includes the step of an analysis of the risk to develop a lupus erythematosus or an analysis of the risk for a relapse of lupus erythematosus in said human.

3. The method of diagnosing lupus erythematosus in a human according to claim 1 or 2, wherein detection of said change of expression comprises a quantitative or qualitative detection method.

4. The method of diagnosing lupus erythematosus in a human according to any of claims 1 to 3, wherein detection of said change of expression comprises using a TREX1 antibody or an RT-PCR.

5. The method of diagnosing lupus erythematosus in a human according to claim 1 or 2, wherein detection of said change of biochemical activity comprises a 3' nucleotide excision assay.

6. The method of diagnosing lupus erythematosus in a human according to claim 1 or 2, wherein said method comprises the steps of:
a) Obtaining a biological test sample from a human to be diagnosed,
b) Analyzing the sequence of the gene encoding for TREX1 in said sample,
c) Comparing said sequence as analyzed with a sequence of the gene encoding for TREX1 in a control sample from a healthy individual, and
d) Diagnosing lupus erythematosus in said human to be diagnosed based on the differences between the two samples,
wherein the presence of a genetic variant (mutation or polymorphism) in the test sample as compared to the control sample is indicative of the risk and/or presence of lupus erythematosus.

7. The method of diagnosing lupus erythematosus in a human according to claim 6, wherein analyzing the sequence of the gene encoding for TREX1 comprises the detection of genetic variants (mutations or polymorphisms) in the regulatory regions, introns and/or exons, in particular the coding exon and/or a sequence according to SEQ ID No. 1, of said gene.

8. The method of diagnosing lupus erythematosus in a human according to claim 6 or 7, wherein detection of said genetic variants (mutations or polymorphisms) comprises at least one of an analysis comprising hybridization, restriction enzyme analysis, and sequencing.

9. The method of diagnosing lupus erythematosus in a human according to any of claims 6 to 8, wherein said genetic variants are selected from the group of polymorphisms g.3829A>T; g.3858A>G; g.4032A>C, rs11925638; g.11170C>T, rs35240314; g.14283G>A; g.14384C>T; g.14455T>C; g.17696C>T, rs34426134; g.18682C>A, rs3135936; g.18743G>A; g.18853A>T; g.20131T>C, rs3135941; g.20570G>A; g.20852C>A; g.21049C>T, rs11797; g.21197G>A; g.21238G>C; g.21257G>C; g.21315A>G; g.21330^ 21331insAA; g.21430G>A, rs3135945; and g.21500T>C, rs3135946.

10. The method of diagnosing lupus erythematosus in a human according to any of claims 1 to 9, further comprising the analysis of other genetic markers for lupus erythematosus, such as MICA, CTLA4, CD154 (3'UTR), PDCD1, RNUX1, PTP22, SCL22A4, SUMO4, and IRF5).

11. The method of diagnosing lupus erythematosus in a human according to any of claims 1 to 10, wherein lupus erythematosus is selected from systemic lupus erythematosis, subacute cutaneous lupus erythematosus, cutaneous lupus erythematosus including chilblain lupus erythematosus, rheumatoid arthritis, Sjogren's syndrome, autoimmune nephritis, autoimmune vasculitis, autoimmune hepatitis, autoimmune carditis, autoimmune encephalitis, autoimmune mediated hematological disease, and other autoimmune diseases.

12. The method of diagnosing lupus erythematosus in a human according to any of claims 1 to 11, wherein said method further includes the step of selecting measures for secondary or primary prevention based on said diagnosis.

13. A method of identifying a compound that modifies the activity of TREX1, said method comprising contacting cells expressing TREX1 with a candidate compound, and identifying said compound by determining the activity of TREX1 in response to said candidate compound.

14. The method of identifying a compound that modifies the activity of TREX1 according to claim 13, wherein the activity of TREX1 is selected from the biochemical activity or the expression of the gene encoding for TREX1.

15. The method of identifying a compound that modifies the activity of TREX1 according to claim 13 or 14, wherein said determining of said change of expression comprises a TREX1 antibody and/or an RT-PCR and/or a 3' nucleotide excision assay.

16. The method of identifying a compound that modifies the activity of TREX1 according to any of claims 13 to 15, wherein said candidate compound is a TREX1 antisense nucleic acid.

17. A diagnostic kit, comprising: a container comprising materials fro performing a diagnostic method according to any of claims 1 to 12, together with suitable agents and instructions for use.

18. A pharmaceutical preparation, comprising a compound as identified according to any of claims 13 to 16, together with a pharmaceutically acceptable carrier and/or excipient.

19. Use of a compound as identified according to any of claims 13 to 16, or a pharmaceutical preparation according to claim 18 for the production of a medicament for the treatment of lupus erythematosus, in particular systemic lupus erythematosis, subacute cutaneous lupus erythematosus, cutaneous lupus erythematosus including chilblain lupus erythematosus, rheumatoid arthritis, Sjogren's syndrome, autoimmune nephritis, autoimmune vasculitis, autoimmune hepatitis, autoimmune carditis, autoimmune encephalitis, autoimmune mediated hematological disease, and other autoimmune diseases.

20. A method of treating lupus erythematosus in a human in need thereof, comprising administering to said human a therapeutically effective amount of a compound as identified according to any of claims 13 to 16, or a pharmaceutical preparation according to claim 18.

21. The method according to claim 20, wherein a second immunosuppressive agent is administered.

22. The method according to claim 20 or 21, wherein said lupus is selected from systemic lupus erythematosis, subacute cutaneous lupus erythematosus, cutaneous lupus erythematosus including chilblain lupus erythematosus, rheumatoid arthritis, Sjogren's syndrome, autoimmune nephritis, autoimmune vasculitis, autoimmune hepatitis, autoimmune carditis, autoimmune encephalitis, autoimmune mediated hematological disease, and other autoimmune diseases.


SEQUENCE LISTING

<110>  Max-Delbrück-Centrum für molekulare Medizin (MDC)

<120>  TREX1 as a marker for lupus erythematosus

<130>  FB17580

<160>  3

<170>  PatentIn version 3.3

<210>  1
<211>  22105
<212>  DNA
<213>  Homo sapiens

<400>  1
ggaaaaaaaa acagtccagc actcagagga atggatgtta acagcctgag cagctcccac      60

cccctcccac acaagaacag ctccaaacat cattcctcct tggactttcc tcctccctgg     120

gcctacctca aatgtacaca tgtgaaacat tcactaaacc cgaagatgtc tgcacacgac     180

agctctattc atactcctca cccaacaaat tccactctcc tcaagaaagc ctaggggttt     240

gtgggaggtg ggtctggcgg cagaaggaaa gcgaggtcac ggagaccaca tccctgaatg     300

atgcctggct cgactcaaac gcaacacgcg tgaatgctag ggcaggctgc tcctgcgcgg     360

ccctcctcca cggagtgcca gggagggagc gcaaagccac cagcgcggcg gagctggtgc     420

gcctccggcc cgcctctccg gtaacgggtc gcgccgcact gagtccttgc agcaaacctg     480

ccgaatcccg cccgtggacc tctaaacacc gggcggggcc gggtgtggcg gcgcgctcgg     540

acgttcgtca ttcctccgcg ccctccgcct gccagctgat tggcccctgg gccaggggcg     600

gggcactcgc ggcggaggca agcggcggcg cgcggacggt tggtccagtt ctccggcctg     660

gcggcaggca agtctagctc ggcgctgtcg gatacttggg gtgagcggaa agcatggcgg     720

ggacctccgc gccaggcagc aagaggcgga gcgagccccc ggcgcctcgc cccggcccgc     780

cgccgggcac cgggcacccc ccgagcaagc gggcccgggg cttctccgca gccgctgccc     840

cggaccctga cgacccgttc ggcgcgcatg gggacttcac tgccgacgac ctggaggagc     900

ttgacaccct cgcgtcacag gccctgagcc aatgtccggc cgcggctcgg gacgtgtcca     960

gtgagtgctc ctcgcggcct tttgctcgga gggagttgtc aaccgcgcca gatccccttg    1020

atggctgtgg cttcggaacc tcgcggccag cactgccttt tcgccttttt aaaatatggg    1080

aacaccctga tttaagcagc ggttgtcttc cagaaggtcc tttgatttta gggggaaatg    1140

cattagccag gtcaaacagc cgatttgaaa cacagaaggc taagttgaca ttttacgtta    1200

ttttctaaag tttaggccac ttggttcttg gttctaagca gagatccttg gaacacacct    1260

accaccacca tcagaaccat cactacccat gcatggggac ccattcttgt gacaagtttg    1320

agcggcttta gatcttatga agcccactgt ctcccttggg cagtgtgagc atgggaggag    1380

ccagacacag gcaagagatg aaataaattt attcttttat acaaccagta aactgtgcta 1440
ggtgcccaaa cgtgaggaac tgcagctgac cttacaatga acaaaataca aaaactgtcc 1500
ttcatggagc ttacattcta gttgaggagt gagtgaagtc ttgaggactc ccagaaagag 1560
aaatgttttg agtgcacctt gcatctgtac ttgggtgtct caaggcacct taaactcaac 1620
acgatcattt cctggaaacc cagtcctcct ccagcacttc tagcatggtg ccggcaaagc 1680
agacattcag agaatgatga ccagatgttc cacaaggaag taactagcac gactctccat 1740
ccagtggcac aaagcgactc ttcccctttc cttgactcta catctaattt atttggaaag 1800
catgtcaagc ttaccttctt aatccttctc tggagtctat cctattcaca ttgcttctac 1860
cagctgcctg atctgtgtta ccatgagtcc tctcttgaac aatggtctgc taaattggtt 1920
tcccatttcc agtcttgact ccctacaagt gaggcttcac atcacattct gagtgatctt 1980
ttcaaaactc aaatctggct gtgtcttaca cagatatacg tgaacaccct tttttttttt 2040
tttttttttt gacggagtct cactctgtca cccaggctga agtgcagtga tgccatcttg 2100
gctcaccaca gcctctacct cccaggttca agagattctc ttgcctcaac ctcccgagta 2160
gcctcagcct cccaagtagc tgggattact tgtgcccgcc accatgcctg gctaattttt 2220
gtatttttag tagagacggg gtttcgccat gttggccagg ctggtctcaa actgctgacc 2280
tcaggtgatc cacccgcctt ggtctcccaa agtgctgaga ttacaggtat gagccatccg 2340
cacccagcca tgaacacccc taatgaaagc cctttaataa tttcttattt cttctagtgt 2400
gaagaccaga atccttaaca tggcctgaaa ggcccagcct ggtttggccc tttctcactc 2460
tctgggtcct ggctgcacta acattctttg aattctctct gcttctttac aatacaggaa 2520
ctttatacat tgttctttct ttgtacattg agtatacttt tcattcattc atttccactc 2580
atctggttaa ttcctacttg tccttcagct ctcagctcct tcaagtcttc cctagggaaa 2640
tgttccaatc atcccagtta agtcagattg ctgtcatatt ataggctttc ctatatttgt 2700
tctcttctgc agttcttaat cacagttgta attttacatt tatctgtgtg attatttgtt 2760
gaatgtcttt taagctctgt gagtgtaagg aagtatcaat tttgctcact attgtacctg 2820
tagagcctaa catgcctggc atattgcatg tggtcagtaa ctattactga ataataagta 2880
gatcattgag ttctggccaa tgagatctcc tcaggcttta gatgagactc ttggaattgg 2940
atgaaggata gggaaagaat aataggatct gttgttttag aaggatcatt tatgagtttc 3000
agccgggtgt ggtggctcat gcctgtaatc ccagcacttt gggaggccaa ggtgggctga 3060
tcacttgagg ccaggagttt gagaccaggc tggccaatat ggtgaaaccc cgtctctact 3120
aaaaatataa aaattagccg ggcgtggtag cacacgcctg taattccagc tacctaggag 3180
ggtgaggcac aagaatcgct tgaacctggg aggtggaggt tgcagtgagc cgagatcacg 3240

ccactgctct ccagcctggg caacagagcg agactctgtc tcaaaaaaaa aaaaaaaaaa 3300
aaaaaaagga tcatttatga gtttgaacca tgttcttagg ttgaaaaaca tctatattgc 3360
ttactagatc tttccctgct ttgctctcaa gagtaattct tcccagcact ttgggaggca 3420
aaggcaggag gattgcttaa gcccaggagt tcaagaccaa cctgggcaac ataaggagac 3480
ccaatctcta caaagaattt taaaattagc caggcatcca ggcacagtgg ctgacgcctg 3540
taatcccaac actttgggag gctgaggcgg gtggatcgcg aggacaggaa atcaagacca 3600
gcctggctaa catggtgaaa cgccatctct actaaaatcc aaaaaaaaaa aaaaaaaaaa 3660
agccaggcat ggtggcacac acctgtagtc ccagctacac aggaggctaa ggcaggagga 3720
tcacttgaac ccaggaggtc aaggtggcag taagcagtga tcacaccact gcactccaac 3780
ctgggtaaca gagcaagacc ctatctcaaa aaagaaaaaa aaaaaaaaag tgggtatttt 3840
cagcattttc tggcaaaagt gggaaaatat tgggtcctga aatgtatatg gaactatttt 3900
ttacaggtga tcataaggtc cacagattat tagatggcat gtcaaaaaat ccttcaggga 3960
aaaacagaga aactgttcca attaaagata atttcgaatt agaggtactt caggcacaat 4020
acaaagaact taaagaaaag gtaagtgact taacttgtgg tttttgtagc taattcattc 4080
acttacgtgt ttaaaagcat tgtacagtaa aatatacaaa agttgtaaaa caagtgtcta 4140
gattcatccc tatgacatgg gaaaagacta gggaaagaaa accagaaagt ttaggctgaa 4200
agaaactaaa ttgtgagatt cctaatgcca aaggcaagaa gaaaaacact gtataccaga 4260
tccaaaaaga gatacaaact gttagggtac taagatgaat ttatagcatg tatcttcatg 4320
tgaataatac aacttcataa tggttgctat tttcattagc agctttgcaa gaaagcagac 4380
acttcatatc accatctgta ggtccctaag aaaaccagga atatgtggaa ttttagttgt 4440
gcaaggtatg tgacccagat tttttttttt tttttttttg gagatggagt ctgtcatcca 4500
ggctggagta cagtgacaca atcttggctc acttcaacct ctgcctcctg gttttaagcg 4560
attcacatgc cttagcttcc agagtagctg ggactacagg tgtgcaccac gacgcccagc 4620
taattcttgt atttttagta gagacggagt ttcaccatgt tggccaggct ggtctcaaac 4680
tcctggcctc agcctcccaa agtgttggga ttacaggcat gagctaccac acccagcgac 4740
ccagatcgtt aatgcctctt agagcctaaa gaatccggcc aggcgcggtg gctcacacct 4800
gtaatcccag cactttggta ggccgaggcc agtggatcac ctgaggtcag gagttcaaga 4860
ccagcctggc caacatggtg aaaacccgtc tctactaaaa atacaaaaat tagctaggca 4920
tggtggcggg cacctgtaat ctcagctact cgggaggctg aggcaggaga attgctggaa 4980
cctgggaggt gaaggttgca gtgaaccgag atcgtgccat tgcactccag cctaggcgac 5040
aatagcaaga ctccgtctca aaaaaaaaaa ggaatctggt caggcaaggt ggctcatgcc 5100
tgtaatccca gcactttggg aggccaaggt gagagaatcg cttgaaccca agagttcaag 5160

accagcctgg gcaatacaat gagaccctgt ctctacagat aaaagtgaaa attggccagg 5220
ctagtggcac atgctttgta gtcctagcta ctcaagaggt tgaggcgaga ggatcacttg 5280
agcccaggag ttcaaggtta cattgagcta tgattgtgcc acagcattcc agcctaagca 5340
accccatccc cctccctggc aaaaagaatc tgcatgcatg agtggcaagt gtatgcttaa 5400
gctatcttga tgaatatcag ctgtcccatg aacaggccta gcagaccatg gtgacatctg 5460
gttgctgtgg atatattttg agtactcatt tgtctgtttt tcctgtgctt tctccatctc 5520
cccaactaca tgttaaaaca aagtacctaa gtagttttct cttacaaagt tttcacgaga 5580
ttaatttggg atttacagat gaaagtaatg gaagaagaag ttctcattaa gaatggagaa 5640
attaaaattt tgcgagactc actacatcag acggaatccg ttctagagga acagagaaga 5700
tcacattttc ttcttgagca agagaaaacc caagcactca gtgacaagga aaaggaattc 5760
tccaaaaagg tgacccagag catttgtttt gcaccagctt tgcctgccca ctgagttcct 5820
ttgaccaggg ttgcctgtaa atcttccagg gagatttcaa cacttgtttg tcttaaatac 5880
tttctgctat catctcattg ccatccactc ttcttccagg gtctggatat attttggaaa 5940
gggatttaga tgaaactcta ttttgctgtg gtagttggtc ttggcaggag taaataacta 6000
gcttagcatt tctagaaaca ggtttacatt aaggctcttt tgctagatca gaaactcaac 6060
tgacctggct taggctaaaa ggaaacgtat tggcttccat atccaagtct gggaagaacc 6120
ggggtatgct gggaccttaa atgacttaaa tcttgccagg acatttttat tctctatgct 6180
ataaacaggc tctatactta tggtagaggg tgtgttctcc agaagctcct gggctcatat 6240
tttataaata cagagcagaa ttaagactta gggccacatg cagtggctca cacctataat 6300
ccagcacttt gggaggctga ggtgagagga ttacttgagc caggagttcg agaccagcct 6360
gggcaactag tgagacctca tctccacaaa aaagaaaaag aaaaaatgaa caaaattatc 6420
caggcatggt ggtgtgcacc tgtagtccca gctactcagg aggctgaggt gggagaattg 6480
cttgagccca ggaggttgag gctgcagtaa gccatgatca tgccattgta ctccagcctg 6540
ggcaatagag cgagaccctg tctcaaaaaa agacttaggc tccctcatct caagttttaa 6600
aattctgaga aaggctctga ttggtttaac ttgagtcagt tgtctgtccc tggccaattg 6660
ctataaccaa aagcaaccaa aagcaaaaaa atgacagctc ccattctaat aaaatagtca 6720
gaggtaggga aattattgaa tttttttttt tttttttttg agacagggtc cagggtttca 6780
ctctcttgcc caggctggag tgcagtggtg caatcacagc tcactgcagc cttgacctcc 6840
caggctcaag caatcctccc atctcagcct cccaggtagc tggaactata ggcacacacc 6900
accaggcaaa gctaattttt aaattttggt acagacaggg ttttgctgta ttctccaagc 6960
cgttctcaag ctcctgggct catgcagtcc tcccaccttg gccttccaaa gtgctgagat 7020

tacaggcgtg agccacaatg cccagcctgt ttctcaggga aaggaaaatg ctattctgga 7080

aggaaaaaca gtaggtatcc agtacctact acaactttcc ctcagcacct cctcatgctt 7140

caatctcaag gacatgggat cttattctca ttgtttcatt cagcaaactt agcaaacttt 7200

aagcacttac ctgtgcccat cattaaccgc aatagttaac tttctgtata agtgatgaag 7260

agcaaggaat ttgcggtcag acaaacctga atttgaatcc aggcttcatc acacataagc 7320

agtgacctca aggtaataac ttaagctccc tggctcattt cccttatctg ttccaagaca 7380

taaccagaaa ggttgttggg agactcacac attcttgcac ttactgtgtc tggtgctggg 7440

cctggcgcag agtgtgcttg gggaatgatg gcagttactg ttggctgtag caactgcagg 7500

ccagagcagc aggtctctct atactgcaca gtgtcttggt aaactattcc aactactctg 7560

cccaaacttt gaggaagacc tggaattgtg caccagtgcg ttcgttaata aaggtcaata 7620

tgttactgct gacatagggc accttaccaa gctctatgct gggtatttta cactcatcat 7680

tctacttaat cctcccaact atcatgaaga caggtgtttt atgttacaaa taagttagga 7740

ttcaaagaca ttaaataaat ggtgggctgg tatttaattc ttttttttat tttttaaatt 7800

ttttgtagag gcaagatctt gctattttgc ccaggctggt ctcgaactcc tgggctcaaa 7860

tgatcctccc gcctcaacct cccaaagtgc ttaggattac aggcctgaac caccacaccc 7920

ggccagggct ggtatttaaa ccctcacaaa ctacccttac aatagagtcc agtatctttt 7980

tagagtacca tattgtcttt cagaaagaaa gctaatgttt aatattttag gacatttatg 8040

tcacagcaga ctttcatatg tatttttcat tgtttgtctt ttactggctt gtgattttga 8100

agcccagaga tatttttgtg tatatgatgg gaccactaca agcatgtttc ttttgccttc 8160

cagctccaat cattgcagtc tgaactccag tttaaagatg cagagatgaa tgaattaagg 8220

acaaagctcc agaccagtga acgagcaaat aaactggctg ctccctctgt ttcccatgtc 8280

aggtaatgat ggtgctggag ggatagttca gttttgcatt atttagtaaa aaatctgcat 8340

aacagtgtca ggctggtcct aaatgaaggc catttagacc cctccacctg ggcagagatc 8400

ctcttttcct gaaggattca gaggtggttt agatgaagct tgcatggttt gagacatcac 8460

agcataaagg cctatactct agaaatgctg cctctgtatg gcactttata aacataattc 8520

ttcaggacta gaaagacctg gaaaaatgat ctccaaattt tgggggtggg ctagtctcta 8580

cttttatgta atttgtttaa ctgattttgt tgcaagcatg ggtgtatttt agttccagct 8640

acttggaata tttttttctc atttgctttc tggggaccct tgtccaacag ccccatcctt 8700

tgtggtcaga tgtgctcaac ttgttttttt tttctttttt ttttgagaca gagtctcgct 8760

ctgtcaccca ggctggagta cagtggtaca atctcggctc actgcaacct ccgcctcctg 8820

ggttcaagca attctcctcc ttcagcctcc tgagtagctg ggactacagg cacacaccac 8880

cacgcccagc taatttttgt atttttagta gagacggagt ttcaccatgt tggccaggct 8940

cgtctcaaac tcctggactc aaatgatttg ccagcctggg cctcccaaac tactgggatt 9000
acaagcatga gccactgtgc ctggcgcagc ctggaaattt gaacagtgtc agtcagggca 9060
tcttctggtt ttgaactttc taccgccttt gttcataatg ggcagtgtgt gaaactgaac 9120
tctccatgcc ttgcattgtc cacataagac aagagaactg aggggatcct cttcacacat 9180
cagtaacaga aagacctcag cattatatga tcagatcaga gggttaatac tgcttctcta 9240
ccataaagac attggcacta agttgctcag ttttcaacat acctagtagg tttatcaaaa 9300
ttatgatatt tattttgata gccctttttc tatttaaagt ctttgcattc tattggaagt 9360
cttttttttt tgagacagag tctcactctg ttgtccaggc tggagtgtag tcacatgatc 9420
tcagctcact gcaacctcca cctcccgggt tcaagtgatt ctcctgtctc agcctcccaa 9480
gtagctggga ttacaggcac ataccaccac accaggctag tttttttgta tttttttttt 9540
aagtagagat gggatttcac catgttggcc aagctgctct cgaactcctg acttcaggtg 9600
atccacccgc ctcagcctcc caaattgttg ggattacaag ggtgagccac tgtgcccagc 9660
ctggaagtct tgtgactatt aaaaaatcgt ttgttccaca ttggctgggt gcagtggctc 9720
acacctgtaa tcccaacact ttgggaggat gaggcgggca gatcacttga ggtcagtagt 9780
tcaagaccaa cctggccaac atgatgaaac cctgtctcta ctgaaaatat aaaaattagc 9840
tgggcatggt ggcatgcgcc tgtaatccca gctatttggg aggctgaggc aggagaatca 9900
ctcaaacctg gaggcagagg ttgcagtgag ccaggatcac accactgcac tccagcctgg 9960
gcaacagaac aagactccat tccagaaaaa aaaatagttt gttccacatt gaaaagatgt 10020
tttgcaccag gcatggtagc tacagaggca atcccagcta cacaggaggc taaggcagga 10080
agattgctta aggctagcag ttcgagacca gcctgggcaa catagcaaaa ccccattgct 10140
acttaaaaaa aaaataaaat tactggcccc agtggctcac gcctgtaatc ccagcatttt 10200
gggaagctga ggtgggcaga tcatttgagc ccacgagttc aggactagcc taggcaacat 10260
ggtgaaactc tatctctaga aataactcta tctctagaaa gaataaaaag ttagccagat 10320
gtggtggttg cacatctgta gtccctgtgc tactcagaag gatgaggtgg gaagatcact 10380
aagcccaaga ggtggaggct gcagtgagcc gtaattgtgc cactgggctc cagcatgggc 10440
aacagagtga gaccctgtct caccaaaaaa aaaaaaaaaa aattagctgg ccatgttgat 10500
gtgtgcctgt aatcccagct actcaggagg ctgggattac agatgggtgg attgcttgag 10560
cccaggaggt caaggctgca gtgagctatg atcatgctac tgtactccag cctgggcaat 10620
ggaacaagac tctgtctcaa aaaaaaatgt tttggtggaa ttgtaaatag tgataataaa 10680
gaatagtaaa ggaattatgg ctaatattat atttttggat gattagaaaa atataaaaat 10740
gagtaaaaga attttcaaca atttggatca aaatccctgg aattagccag taacagtgga 10800

tcatgcctgt aatcccagca ttttgggagg ccagagtggg tggatcactt gagctcagca 10860

gttccagacc agcctgggta acttagtgaa tccccatctc tatagagaaa ttaaaattta 10920

aaaatatttt taaaaagaaa aacaaatgcc tggaattttg cactgctcct ctgacaaaga 10980

atggtagaca tataatagat aaaacctttg gttaaaacac ctagacatga aaagtttaaa 11040

gtttgttaga aattttgtaa ccagatattt tgaatggttt tagtagaatc attactttgc 11100

tttcatagtt aactttttcc agtcctagga aaaacccttc tgtggttata aagccagaag 11160

catgttctcc acaatttgga aaaacatctt ttcctacaaa ggagtctttt agtgctaaca 11220

tgtcccttcc ccacccctgc cagacggagt caggatacaa gcctctggtg ggcagagagg 11280

gtaagtccat tagtcatcta ttgatgtata acaagctacc caaatatgtg gcttaaaaca 11340

attattattt attttctccc aatttctgtg atcagcaatt ctgacaaggc acagagggga 11400

caatctctgc agggactgga atcactgaaa gcttgtctta agctggagga tctatttcaa 11460

ggtggctcac tcacatggct ggcagtgtaa tttcttgcca catgggcctc tgcacatgga 11520

tatggggtat cctcttaacc tggtgggtgg cttcctctaa agctagcaaa tccagagaga 11580

gcgagccagg cagaagctat ccatttatgg cctagcctca gaagtcacac agcatcccct 11640

ctgccacatt gtatttgtta gaagcaaatc actgagtctg ggcgtcattc aaggggagaa 11700

accaagctta tacctttttt ttaattttta tttttatttt ttattttatt attatacttt 11760

aaattttagg gtacatgtgc acaacgtgca ggtgtgttac gtatgtatac atgtgccatg 11820

ttggtgtgtt gcacccatta actcgtcatt tagcattagg tatatctcct aatgctatcc 11880

ctcccccctc ctcccaagct tatacctttt gaaggggagt tgtgtcaaag catttgtggg 11940

cctttttttt tttttttttg aggcggagtc tcaccctgtc acccaggctg gagtgcaatg 12000

gcacgatctc agctcactgc aacttccgcc tcccgggttc aagtgattct cctgcctcag 12060

actcccgaat agctgggatt acaggcacct gccatcatgc ttgtgggcat atttttaaac 12120

catcacagca tcaattgacc atgccaaaaa cctagctagt atgcaggaat ctgtttttct 12180

tttcttgttt tttttttttt ctttgaggcg gagtctcact ctgtcaccag gctggagtgc 12240

agtggcgtga tctcggctca ctgtaacctc tgcctcccat gttcaagcga ttctcctgcc 12300

tcagcctccc gagtagctgg gactacaggc atgcgccacc acacccagct aatttttgta 12360

tttttagtag agacaggttt caccatgttg gcctggatgg tctcaatctc atgacctcgt 12420

gatccaccca cttcagcctc ccaaaatgct gggattacag gcatgagcca ccgtgcccag 12480

cctctgtttt ccttttgtag tccagctttt gagtttttat aactcagtcc agattgaggg 12540

gatctgctgt tttttagaga gagacaatct aacatcagta ggttttcaga ctgctatgtc 12600

agtgtatctt ataaatcgga tacattgacc ctcttcttac catcaacctt tcaaatcctt 12660

ggccatcata acacttctga gaaactgtag cccacttaca tttttggcat ctagcagcta 12720

aaggggcagt gtacagtagt gttacaagcg ggcagtagag cccagtagcc tggatgtgag 12780

tcccagtaat gctgttaact ggcttatatg atttgggcaa gtcctttagt ctctgtgcct 12840

cagtttccct atatgtaaaa cagacctaat gataaatcct tgtagggtca ctgaggaaca 12900

aatgggctga tgcgtataga gctcttaaaa cactgtctag cacgtaacaa gcaccatgtc 12960

aatgttagtg gtcactgttt tatctaattg cctcattttg agtgaggcaa acagcttgat 13020

tttcttttcc tttccttgta tgagttcctg ttttacataa ggaaggtaaa agatgcaggg 13080

ctcttgagcc gctttggctc ttgtttgttc caagttcgaa gaagtagaac agcgtgtgtt 13140

ttaaactcat tgcatgtaaa agtttctaat atgcccatgc ttctgggctc aagttttgat 13200

ttacatttta tcacatttca gatagtaagc cccacagtct gagaggtgac tccataaaac 13260

aagaagaggc ccagaaaagc tttgttgaca gctggagaca gagatcaaac actcaaggta 13320

ccagaatccc tgcttctcct gcaggggcct gcatggctct gagtaatatg cagaagaatt 13380

gcccaggcct ctgagaaccg gtgccccggg cagtccttca gagagtccag ggaagtcaaa 13440

gtatgtggct taagcagaag actttataaa gtggaaaggg ccaagggcag gtatgtccca 13500

gagccttccg cacccacagg catcttcctg gggcttagtg gcagcctaga ggacagttgg 13560

catccaaaga atccttactg tttccttctg aaagccaccg aaaagatctc ccatgtcaga 13620

accttctaga gtcatcttgt cttctgcagg ttccattttg ataaacctgc tcctgaagca 13680

gcctttgatc ccagggtcat ccctaagcct ttgccacctc ctgagtagta gttctgagtc 13740

tcctgctggc acccccctgc agccaccagg gtttggcagg taagcataag actcctggaa 13800

agcttgtttg ggaaggagct tcctggaagc aaattccttt ctggccctgg ccagcctgag 13860

aagtctgtgg gaagggcagg cagcctggag aatttggcag gcaggctgtt tggggctctt 13920

gaccttatct ccatcccaca cttaatctat ttttctttgt gtttgttgcc agtaccttgg 13980

ctggaatgtc aggcctcagg accacaggtt cttatgatgg gtcattttcc ctctcagccc 14040

tgagagaagc acagaacctg gcattcactg gactgaatct ggttgcccgg aatgagtgct 14100

cacgtgatgg agacccagca gagggaggca gaagggcctt cccactctgc cagcttcctg 14160

gagccgtgca tttcctcccc cttgtacagt tcttcatcgg cttacactgc caggccctgc 14220

aggacttggc agctgctaag agaagcggag cacctgggga ctcaccgaca cattcctcct 14280

gcgtgagctc tggggtagag accaaccctg aggactcagt gtgcatcctg gaaggcttct 14340

ctgtgactgc acttagcatt cttcagcacc tggtgtgcca cagcggagca gtcgtctccc 14400

tattactgtc aggagtgggg gcagattctg ctgctgggga aggaaacagg agcctggttc 14460

acaggcttag tgatggagat atgacctcag ccctaagggg ggttgctgat gaccaaggac 14520

agcacccact gttgaagatg cttcttcacc tgttggcttt ctcttctgca gcaacaggtc 14580

accttcaagc cagtgtcctg acccagtgcc ttaaggtttt ggtgaaatta gccgaaaaca 14640

cttcctgtga tttcttgccc aggtattaag ctgcatagga gtcatgattc tttgtgggtc 14700

tcacctgacc tcttaaggtc taacccatgg cacttgtact ttgctcacat cttgacttat 14760

ctacactagt tagttctaag gcttggttca gggagttctg aagtgatggg aagtggggcc 14820

aaatgggaga accaaggaag tggtttattt gatttaattc tgcctctgaa caacaggtaa 14880

gcaaaaggca cacatgatta aaggaggctg ctaacctctg tggaggaggg ccctgcccca 14940

ggacaagggt taagacaagg ggcctctgaa cctgagatgt gggtaaggtg aagaccacac 15000

atcacaagca cgttgaagtt tagtggtttc cctaaggaag cagaggatgt ggagagggtc 15060

tgcagcccac gggctgacct tgctgggagt ctctgcctgt cattgtgccc atctttagca 15120

tgcatctggg gtggcctgtt cttcttaagg tttgtgggta gtatattata gtgaacctag 15180

cgaatgaaca ctgtgatcat aaattcctcc cttgggattc cagagccgca agattgggga 15240

gaactgtgaa cacaaccagc ccaccccctc atccagtaat ccagtctccc tcagcactct 15300

accaaagggt cagtttctat gttggaatga ttccactggc tgaatatatc ttaaatcatc 15360

tcatctctga gctcagcact gctgtttcct agatctgttt ccatctgaat atttttggtt 15420

ggtctggccc atgctaaagg tggtactcag ctggacacag cactgcagtg tagtgtgggc 15480

agtgggatca ggcagactca ggactcaagt gcccagcagg ggcccagctc atagtggctg 15540

tttttttttg ttgttgttta tttgtttgtt tttgtttttg ttttgagacg aagttttgct 15600

cttgttgtcc aagctggagt gcagtggtgc gatctcggct cactgcaacc tccacttccc 15660

aggttcaagc agttctcctg cctcagcctc ctgagacgct gagattacag gcacctgcca 15720

ccacacctgg ctaatttttt atatttttag tagagacagg gtttcaccat gttggccagg 15780

cttgtctcaa atgccggacc tcaggtgatc cacctgcctt ggcctgccaa agtgcaagga 15840

ttacaggcgt gagccaccgg gcccagcctt ggttatttgt ttaataattg ttaaatgaat 15900

gaatcccagg ctgggcacag tggctcctgc ctgtaatctc agcactttgg gaggccaagg 15960

tgggaggatc acttgagccc aggagtttga gaccagcgtg ggcaacaaag caagactccc 16020

agctctacaa tttttttttt tttaattagc caggcatggt ggcagtgagc tatgaccatg 16080

ccactgcact ccagcctagg tgacagagtg agaccctatc tcaaagaatc ccagaaacca 16140

tgtttttact ggtggtagac acaccattat tccactcctg ttgagcttgt ccacagggac 16200

attcaaaaca cagccacagc tgggcattta tctatttcat gtactggggc tcttcataaa 16260

atttcattta aagaggttct taggggctgg gcatggtggc tcacgcctgt aatcccagca 16320

ctttaggaag ccgaggtggg tggatcacga ggtcaggaga tcgagaccat cctggctaac 16380

atggtgaaac cccgtctcta ctaaaaatac aaaaaagtag ctgggcgtgg tggcaggcac 16440

ctgtagtccc agctactcgg gaggctgagg caggagaatc gcttgaaccc tggaggcgga 16500

ggttgcagtg agctgagttt gcgccactgc actccagcct gggtgacaga gcgagactca 16560
gtttcaaaaa aaaaaagttc ttaacgctat caaaaaataa cttggaaacc actgtggtgt 16620
acataaaagt ggattttcct tgcagtacct tctgcaagtg gcagatattg gacatcttac 16680
agcccaataa tgaaaccagg taataaccat gttaaaattc cagcttccca caaagggctg 16740
cacaccctgg caactaaaaa gcattgaact ttttgtctgc tgtgcaggat ttgccctaaa 16800
gtgagttcac cccacatagt gagaccttgc ttcctaagga cacactgagg gatatcttga 16860
ccttctgtgc ttagaaactg cagatgtctg aaggggtaca cacctggcca gaccccatag 16920
agtgtttcag aattgccctc ctccccaccc accaggccag tccatcgtgc tcccccaggc 16980
tatgtgggtt gagcaaacag cagcagttgc cttggggctt ggtttattgg ggtaatagct 17040
ttccttttgc ccctgagacc tggggcagta tgcccagaac ccctccctcc tgcacacccc 17100
acagtaggca cattctccca ctggcagcag atgtgttttc tggcaagtag agaaaatggc 17160
tttccttgcc ctgtgagagg tctcataaga tccagccata gccaaggcca tgtttgtcac 17220
ctctggaggc ctggtcagtc cttctgtggg cacagaacat ttctctccca cctaagttag 17280
gacctgcatc cctgtttggg agcatgtgga agccaggcat cccagatcca ggaggtcatc 17340
ccacttcctg ggcgatgcta caccagataa gggcctgtcc tctcagctgc agcagtcttg 17400
cagtggaagt gcccacttgt tcacatgaaa caagtttcat agcaagtaag taggggtcag 17460
gccagcactg aaacccaagt ctggctgaac caactcacct gttcatactg acttgctcaa 17520
ttttctgtta cctctagtgg agtgtgaagg taggttatgg agctggggtt ggggagtgtc 17580
acgtctctct gggtccctgt cttttaggtt ccagtgtgtg ttccaagtgc tgccaaagtg 17640
cctcagccca gagacacccc tgcctagcgt gctgctggct gttgagctcc tctccctgct 17700
ggcggaccac gaccagctgg cacctcagct ctgttcccac tcaggtaaag cagggtgggg 17760
cgggcatcta gactgctcct gcagatcaag ggaagggttg gggtgggaac agggtcaagg 17820
gcctcagagg gcttctgcag tatgtagccc acgctcttta tgagaaaggg caccctgagt 17880
gagaggtgcg ctgtcctttt cagaaggctg cctcctgctg ctgctgtaca tgtacatcac 17940
atcacggcct gacagagtgg ccttggagac acaatggctc cagctggaac aagaggtaaa 18000
aactccagag ccccttctgg acactgtccc caccccatcc taagaaccaa cagaatctcc 18060
tttctttccg ctgaggagaa acggagcttt aattcatttt agtaacaaga gttgataaag 18120
caactaaaga ggtaaactca gtttgggggc tgtgcaccac acagatgcaa tgcagacagg 18180
acttggcaga gttgcctgat gactgatcct gtcttggctt tgggacgtgg gggcagaata 18240
gggggctgga ggcaggtggg ttgctggtga cacatgagtg cttgtcctgg gtcttccctg 18300
ggaaggtccc acaccacttc ttggacccca tttgtgcaga caaacagttg ctgaagcaag 18360

tgaactctta tagaagctta agaaaaccct cgcagggact ggtctccttc tgcccaggat 18420

ggaaccactc tgttcttgtt ctaggtggtg tggctcctgg ctaagcttgg tgtgcagagc 18480

cccttgcccc cagtcactgg ctccaactgc cagtgtaatg tggaggtgag tgggtagggg 18540

ccaacagctg gcagctctgg tggtaagggg gccccgtgca aggactgtag gccccactgc 18600

aaaccccctc acgtgaggtg gctaggctga gcggattgtt agggtgcagg ccatggtggc 18660

accaggcctc agtctgcacc ccccctctct caggtggtca gagcgctcac ggtgatgttg 18720

cacagacagt ggctgacagt gcggagggca gggggacccc caaggaccga ccagcagagg 18780

cggacagtgc gctgtctgcg ggacacggtg ctgctgctgc acggcctatc gcagaaggac 18840

aagctcttca tgatgcactg cgtggaggtc ctgcatcagt ttgaccaggt gatgccgggg 18900

gtcagcatgc tcatccgagg gcttcctgat gtgacggact gtgaaggtaa gcctgccaga 18960

ggccatcctg cccagccccc atggcttctt ccagaggttc cccaacaagt cagattcctg 19020

ggtgtcccct cagcaggccc ccgcccactg cagcctgttc cagcactggg gccattttct 19080

tttatcttcc tgcctcagtt cttctccaag catattggga tgccttttgc atctatctgt 19140

tgagtgtgcc tggcagagcc acggtttttc cagaaatagc cgtgtctgaa ttgcctttgt 19200

atcactttgt ttgcagtagc tgagggagca gggcctgggt gtggaaggga ctggttagtt 19260

cctgcggacg ttgggggagg agaggtggga cctgccctag gccctggcac ctttgggccc 19320

tcaccaggaa cctctccttt ttgtctcaga ggcagccctg gatgacctct gtgccgcgga 19380

aaccgatgtg gaagaccccg aggtggagtg tggctgaggc cctgagtgtc cagccacatg 19440

gtggcaccag caccactcct ttccttacca catcaactga ttaaagcagt gaccagcagg 19500

aactgcccag agaactggct ggccttgttt cctgagtctg atctgtttgg cggagtggga 19560

ggggtggagc aggacccgga ccctgagtgg ctgggatcct tcttcctgtc cctggctgtt 19620

gctgagcccg tccccatggt aactgatctg ccttgaggaa ggagccctgc cctgcctgtg 19680

gaattgtcct gagtcattgc tttgggctgg ggccatggga agaaaccatt gtgtggcagg 19740

gaaggaggtg gctcttggcc caggcctaaa ccaggaaagc ctgggaaact gggacccaca 19800

ggtgggcatg aaagggccgc agcaggggct cccagcagtg tgtaagaccg ggagctggtc 19860

tggcaccact gccctggtcc ttccagctgc ctgtcactgg tatgatggcc ccggtgcatt 19920

gtgccaccag caggccacag ctgtggatct tggaaggcct ctggggtccc ccgggagcag 19980

gggagtgggt gtggggggga acggatggtg gtgagaggga cagaccaggc aggctgacga 20040

gcagggcggg cctggctcac gtgggcctgt aggcgggccc acgccaagtt tcacttcccg 20100

ccactgctgc cagcgagagc cgcgggagag tgtgcagccg agtcactact gcctgcctgc 20160

ctgcctgcta cggtgagtgt ggcccccaca atgggatggc gcagggcagg agggccatgg 20220

gttcccccac cccagactaa gggggcacta ggggaggggc cgagtcatgt gaagagggag 20280

accctctcag acagtcgaat gtgctggtcc cactaaggaa accacctcac cctctccaac 20340
ttcctgcctg aaaatgggcc ctggagctcg cagacagggc aggattgtgc agggaaggcc 20400
tgagatgtgc ttctgcccac cccctacccc actccctccc cttcggatct taacactggg 20460
cactcacaca cccaccccat gctcctctcc aggctcagca gcaggtacgt acccaaccat 20520
gggctcgcag gccctgcccc cggggcccat gcagaccctc atctttttcg acatggaggc 20580
cactggcttg cccttctccc agcccaaggt cacggagctg tgcctgctgg ctgtccacag 20640
atgtgccctg gagagccccc ccacctctca ggggccacct cccacagttc ctccaccacc 20700
gcgtgtggta gacaagctct ccctgtgtgt ggctccgggg aaggcctgca gccctgcagc 20760
cagcgagatc acaggtctga gcacagctgt gctggcagcg catgggcgtc aatgttttga 20820
tgacaacctg gccaacctgc tcctagcctt cctgcggcgc cagccacagc cctggtgcct 20880
ggtggcacac aatggtgacc gctacgactt ccccctgctc caagcagagc tggctatgct 20940
gggcctcacc agtgctctgg atggtgcctt ctgtgtggat agcatcactg cgctgaaggc 21000
cctggagcga gcaagcagcc cctcagaaca cggcccaagg aagagctaca gcctaggcag 21060
catctacact cgcctgtatg ggcagtcccc tccagactcg cacacggctg agggtgatgt 21120
cctggccctg ctcagcatct gtcagtggag accacaggcc ctgctgcggt gggtggatgc 21180
tcacgccagg cctttcggca ccatcaggcc catgtatggg gtcacagcct ctgctaggac 21240
caagccaaga ccatctgctg tcacaaccac tgcacacctg gccacaacca ggaacactag 21300
tcccagcctt ggagagagca ggggtaccaa ggatcttcct ccagtgaagg accctggagc 21360
cctatccagg gaggggctgc tggccccact gggtctgctg gccatcctga ccttggcagt 21420
agccacactg tatggactat ccctggccac acctggggag taggccaaga aggaaaatct 21480
gacgaataaa gacccccgct gccccatagc actgagtggt caattggctc ctacccttct 21540
tggcagcaca gacctctgcc cacacccagg gacctgggga ggagggcaca gtgtttgtcc 21600
ctcactttcc ctagcaggat ggtcaactat ctgagcccag taaaagtagg gaaaacacag 21660
agaagcccaa atggatggaa agagtacagc ctgattccaa ttccagcagc cgaggccagg 21720
aagaaagggg gcagcagctc caaacgattg catttattat aaacaagtgt acagacccta 21780
gactcagaaa cacaacagat ttgagctaag acagctctgg tgaaacagta atagagggag 21840
gaggaacacg aggtattcat gtctgggcca gagctgccat ccagggcccc acaccccacc 21900
tttggtccag atggcccatt ggcccaggtg tccctgctgc cagaacaccg tggactgggg 21960
tacaggagtt gttgcatatt ccatgaggct ggtgtcggga agcagggact cacagttgcc 22020
aggttgtcca tctctgagcc aatttccctc cacaacccag gggtttcagt ctcatatcaa 22080
ctatcatgtt tgaaacagaa aacag 22105

<210>  2
<211>  314
<212>  PRT
<213>  Homo sapiens

<400>  2

<210>  3
<211>  3582
<212>  DNA
<213>  Homo sapiens

<400>  3
   tgcagtctga actccagttt aaagatgcag agatgaatga attaaggaca aagctccaga      60
   ccagtgaacg agcaaataaa ctggctgctc cctctgtttc ccatgtcagt cctaggaaaa     120
   acccttctgt ggttataaag ccagaagcat gttctccaca atttggaaaa acatcttttc     180
   ctacaaagga gtcttttagt gctaacatgt cccttcccca cccctgccag acggagtcag     240
   gatacaagcc tctggtgggc agagaggata gtaagcccca cagtctgaga ggtgactcca     300
   taaaacaaga agaggcccag aaaagctttg ttgacagctg gagacagaga tcaaacactc     360
   aaggttccat tttgataaac ctgctcctga agcagccttt gatcccaggg tcatccctaa     420
   gcctttgcca cctcctgagt agtagttctg agtctcctgc tggcaccccc ctgcagccac     480
   cagggtttgg cagtaccttg gctggaatgt caggcctcag gaccacaggt tcttatgatg     540
   ggtcattttc cctctcagcc ctgagagaag cacagaacct ggcattcact ggactgaatc     600
   tggttgcccg gaatgagtgc tcacgtgatg gagacccagc agagggaggc agaagggcct     660
   tcccactctg ccagcttcct ggagccgtgc atttcctccc ccttgtacag ttcttcatcg     720
   gcttacactg ccaggccctg caggacttgg cagctgctaa gagaagcgga gcacctgggg     780
   actcaccgac acattcctcc tgcgtgagct ctggggtaga gaccaaccct gaggactcag     840
   tgtgcatcct ggaaggcttc tctgtgactg cacttagcat tcttcagcac ctggtgtgcc     900
   acagcggagc agtcgtctcc ctattactgt caggagtggg ggcagattct gctgctgggg     960
   aaggaaacag gagcctggtt cacaggctta gtgatggaga tatgacctca gccctaaggg 1020
   gggttgctga tgaccaagga cagcacccac tgttgaagat gcttcttcac ctgttggctt 1080
   tctcttctgc agcaacaggt caccttcaag ccagtgtcct gacccagtgc cttaaggttt 1140
   tggtgaaatt agccgaaaac acttcctgtg atttcttgcc caggttccag tgtgtgttcc 1200
   aagtgctgcc aaagtgcctc agcccagaga cacccctgcc tagcgtgctg ctggctgttg 1260
   agctcctctc cctgctggcg gaccacgacc agctggcacc tcagctctgt tcccactcag 1320
   aaggctgcct cctgctgctg ctgtacatgt acatcacatc acggcctgac agagtggcct 1380
   tggagacaca atggctccag ctggaacaag aggtggtgtg gctcctggct aagcttggtg 1440
   tgcagagccc cttgccccca gtcactggct ccaactgcca gtgtaatgtg gaggtggtca 1500
   gagcgctcac ggtgatgttg cacagacagt ggctgacagt gcggagggca gggggacccc 1560
   caaggaccga ccagcagagg cggacagtgc gctgtctgcg ggacacggtg ctgctgctgc 1620
   acggcctatc gcagaaggac aagctcttca tgatgcactg cgtggaggtc ctgcatcagt 1680
   ttgaccaggt gatgccgggg gtcagcatgc tcatccgagg gcttcctgat gtgacggact 1740
   gtgaagaggc agccctggat gacctctgtg ccgcggaaac cgatgtggaa gaccccgagg 1800
   tggagtgtgg ctgaggccct gagtgtccag ccacatggtg gcaccagcac cactcctttc 1860
   cttaccacat caactgatta aagcagtgac cagcaggaac tgcccagaga actggctggc 1920
   cttgtttcct gagtctgatc tgtttggcgg agtgggaggg gtggagcagg acccggaccc 1980
   tgagtggctg ggatccttct tcctgtccct ggctgttgct gagcccgtcc ccatggtaac 2040
   tgatctgcct tgaggaagga gccctgccct gcctgtggaa ttgtcctgag tcattgcttt 2100
   gggctggggc catgggaaga aaccattgtg tggcagggaa ggaggtggct cttggcccag 2160
   gcctaaacca ggaaagcctg ggaaactggg acccacaggt gggcatgaaa gggccgcagc 2220
   aggggctccc agcagtgtgt aagaccggga gctggtctgg caccactgcc ctggtccttc 2280
   cagctgcctg tcactggtat gatggccccg gtgcattgtg ccaccagcag gccacagctg 2340
   tggatcttgg aaggcctctg ggtccccccg ggagcagggg agtgggtgtg ggggggaacg 2400
   gatggtggtg agagggacag accaggcagg ctgacgagca gggcgggcct ggctcacgtg 2460
   ggcctgtagg cgggcccacg ccaagtttca cttcccgcca ctgctgccag cgagagccgc 2520
   gggagagtgt gcagccgagt cactactgcc tgcctgcctg cctgctacgg ctcagcagca 2580
   ggtacgtacc caaccatggg ctcgcaggcc ctgcccccgg ggcccatgca gaccctcatc 2640
   tttttcgaca tggaggccac tggcttgccc ttctcccagc ccaaggtcac ggagctgtgc 2700
   ctgctggctg tccacagatg tgccctggag agccccccca cctctcaggg gccacctccc 2760
   acagttcctc caccaccgcg tgtggtagac aagctctccc tgtgtgtggc tccggggaag 2820
   gcctgcagcc ctgcagccag cgagatcaca ggtctgagca cagctgtgct ggcagcgcat 2880
   gggcgtcaat gttttgatga caacctggcc aacctgctcc tagccttcct gcggcgccag 2940
   ccacagccct ggtgcctggt ggcacacaat ggtgaccgct acgacttccc cctgctccaa 3000
   gcagagctgg ctatgctggg cctcaccagt gctctggatg gtgccttctg tgtggatagc 3060
   atcactgcgc tgaaggccct ggagcgagca agcagcccct cagaacacgg cccaaggaag 3120
   agctacagcc taggcagcat ctacactcgc ctgtatgggc agtcccctcc agactcgcac 3180
   acggctgagg gtgatgtcct ggccctgctc agcatctgtc agtggagacc acaggccctg 3240
   ctgcggtggg tggatgctca cgccaggcct ttcggcacca tcaggcccat gtatggggtc 3300
   acagcctctg ctaggaccaa gccaagacca tctgctgtca caaccactgc acacctggcc 3360
   acaaccagga acactagtcc cagccttgga gagagcaggg gtaccaagga tcttcctcca 3420
   gtgaaggacc ctggagccct atccagggag gggctgctgg ccccactggg tctgctggcc 3480
   atcctgacct tggcagtagc cacactgtat ggactatccc tggccacacc tggggagtag 3540
   gccaagaagg aaaatctgac gaataaagac ccccgctgcc cc 3582